# EUROPEAN PATENT APPLICATION

(11) **EP 3 158 997 A1**
(43) Date of publication of application: **26.04.2017**
(21) Application number: 14894904.3
(22) Date of filing: 17.12.2014
(51) Int. Cl.: A61K 31/496, A61K 31/4985, A61K 36/18, A61K 36/48, A61P 3/10

(54) **COMPOSITION WITH HIGH CONTENT OF CYCLIC DIPEPTIDE**

(30) Priority: 20.06.2014 JP 2014127768
(71) Applicant: Suntory Holdings Limited, Osaka-shi, Osaka 530-8203 (JP)
(72) Inventor: SUZUKI, Toshihide, Mishima-gun Osaka 618-8503 (JP); YAMAMOTO, Kenji, Mishima-gun Osaka 618-8503 (JP); BEPPU, Yoshinori, Mishima-gun Osaka 618-0001 (JP); WATANABE, Hiroshi, Mishima-gun Osaka 618-8503 (JP)
(74) Representative: Vossius & Partner Patentanwälte Rechtsanwälte mbB
(86) International application number: PCT/JP2014/083467
(87) International publication number: WO 2015/194070

(57) **Abstract**

Provided is a composition having an excellent physiological effect. Inventors have found that a composition in which the total amount of cyclic dipeptides including amino acids as constituent units, or salts thereof, is greater than or equal to a specific amount has the effect of improving sugar metabolism, and have also found that the sugar metabolism improvement effect is achieved by including given contents of specific cyclic dipeptides. This composition is advantageous in that it has an excellent sugar metabolism improvement effect, and that it can be taken in safely for a long period of time.

## Description

### TECHNICAL FIELD

The present invention relates to a composition with a high content of a cyclic dipeptide including amino acids as constituent units.

### BACKGROUND ART

"Dipeptides", which are each composed of two amino acids bonded to each other, have been paid attention as functional substances. Dipeptides can be provided with physical properties or novel functions that are not possessed by simple amino acids and are expected as materials having application ranges broader than those of amino acids. In particular, diketopiperazines, which are cyclic dipeptides, are known to have various physiological activities, and demands for diketopiperazines are predicted to increase in the medical and pharmacological fields.

For example, Patent Literature 1 reports that cyclic dipeptides having 2,5-diketopiperazine structure have an antidepressant action, a learning motivation-improving action, etc. Non Patent Literature 1 discloses that the cyclic dipeptide Cycle(His-Pro) has various physiological activities including central nervous system actions such as decrease in body temperature and appetite suppression and hormone-like actions such as suppression of prolactin secretion and acceleration of growth hormone secretion, and the literature also reports that the cyclic dipeptide Cyclo(Leu-Gly) has a memory function-improving action and the cyclic dipeptide Cyclo(Asp-Pro) has a suppressing action on preference for fat. Non Patent Literature 2 reports cyclic dipeptides having an antibacterial action or an antioxidant action.

Non Patent Literature 3 reports that the cyclic dipeptide Cyclo(Trp-Pro) has an anticancer action, the cyclic dipeptides Cyclo(His-Pro) and Cyclo(Gly-Pro) have an antibacterial action, the cyclic dipeptide Cyclo(His-Pro) has a neuroprotective action, the cyclic dipeptide Cyclo(Gly-Pro) has a memory function-improving action, and the cyclic dipeptides Cycle(Tyr-Pro) and Cyclo(Phe-Pro) have a biological herbicide action. Regarding effects of cyclic dipeptides on glucose metabolism, it is reported that cyclo-histidyl-proline (Cycle(His-Pro)), which is one of cyclic dipeptides and obtained by treating a yeast suspension with protease, increases glucose tolerance and Cyclo(His-Pro) has an antioxidant activity (Non Patent Literature 4).

The glucagon-like peptide (GLP-1), which is known as a weight-loss hormone, is an incretin consisting of 30 or 31 amino acids, and is released from enteroendocrine L cells in response to fats and oils, uptake of carbohydrates, and proteins derived from diet. It has been found that release of the peptide hormone decreases in individuals with Type II diabetes and acceleration of GLP-1 release in Type II diabetes is considered to be effective for treatment of diabetes and other related diseases (Non Patent Literature 5). In addition, the GLP-1 is known to have a function of appetite suppression through increase in insulin secretion in response to uptake of carbohydrates (uptake of glucose) to work on a specific area in the brain in normal condition (Non Patent Literature 6).

### CITATION LIST

### PATENT LITERATURE

Patent Literature 1: National Publication of International Patent Application No. 2012-517998

### NON PATENT LITERATURE

Non Patent Literature 1: Peptides, 16(1), 151-164 (1995)
Non Patent Literature 2: Bioscience & Industry, 60(7), 454-457 (2002)
Non Patent Literature 3: Chemical Reviews, 112, 3641-3716 (2012)
Non Patent Literature 4: J of Food Science, vol 76(2) 2011
Non Patent Literature 5: Nauck et al., 1993, J Clin Invest. 1993 Jan; 91(1):301-7
Non Patent Literature 6: Zander et al., 2002, Lancet, 359, 824-830 (2002)

### SUMMARY OF INVENTION

### TECHNICAL PROBLEM

Although cyclic dipeptides are reported to have a physiological activity, they possibly have still unknown functions.

It is an object of the present invention to provide a composition having a preferred physiological action.

### SOLUTION TO PROBLEM

The present inventors, who have diligently studied the action of cyclic dipeptides, have found that a composition containing a cyclic dipeptide including amino acids as constituent units or a salt thereof with a high content, i.e., with its total amount being higher than or equal to a specific amount, has an excellent glucose metabolism-ameliorating action, and further found that a specific cyclic dipeptide contained at a predetermined content allows the glucose metabolism-ameliorating action to be exerted, and have arrived at the completion of the present invention. Specifically, the present invention provides the following aspects:
1) A composition containing a cyclic dipeptide including amino acids as constituent units or a salt thereof, wherein the total amount of the cyclic dipeptide including amino acids as constituent units or salt thereof is 0.2 × 10 to 1.0 × 10⁶ ppm.
2) The composition according to aspect 1), wherein the cyclic dipeptide including amino acids as constituent units or salt thereof is one or two or more cyclic dipeptides or salts thereof of the following (1) to (71) each contained in an amount satisfying the content range:
   (1) cyclo-threonyl-tyrosine or a salt thereof, content: 0.40 x 10 to 0.70 × 10⁵ ppm;
   (2) cyclo-valyl-tyrosine or a salt thereof, content: 0.30 × 10 to 0.50 × 10⁵ ppm;
   (3) cyclo-glutaminyl-tyrosine or a salt thereof, content 0.20 × 10 to 0.40 × 10⁵ ppm;
   (4) cyclo-asparaginyl-isoleucine or a salt thereof, content: 0.40 × 10 to 0.70 × 10⁵ ppm;
   (5) cyclo-arginyl-proline or a salt thereof, content: 0.40 × 10 to 0.70 × 10⁵ ppm;
   (6) cyclo-asparaginyl-glycine or a salt thereof, content: 0.20 × 10 to 0.50 × 10⁵ ppm;
   (7) cyclo-methionyl-valine or a salt thereof, content: 0.40 × 10 to 0.70 × 10⁵ ppm;
   (8) cyclo-glutamyl-cysteine or a salt thereof, content: 0.40 × 10 to 0.70 0 × 10⁵ ppm;
   (9) cyclo-seryl-glutamic acid or a salt thereof, content: 0.40 × 10 to 0.70 × 10⁵ ppm;
   (10) cyclo-isoleucyl-lysine or a salt thereof, content: 0.50 × 10 to 0.90 × 10⁵ ppm;
   (11) cyclo-glutaminyl-leucine or a salt thereof, content: 0.80 × 10 to 1.30 × 10⁵ ppm;
   (12) cyclo-isoleucyl-threonine or a salt thereof, content: 0.20 × 10 to 0.50 × 10⁵ ppm;
   (13) cyclo-threonyl-threonine or a salt thereof, content: 0.30 × 10 to 0.50 × 10⁵ ppm;
   (14) cyclo-methionyl-threonine or a salt thereof, content: 0.40 × 10 to 0.70 × 10⁵ ppm;
   (15) cyclo-alanyl-cysteine or a salt thereof, content: 0.40 × 10 to 0.70 × 10⁵ ppm;
   (16) cyclo-glycyl-cysteine or a salt thereof, content: 0.40 × 10 to 0.70 × 10⁵ ppm;
   (17) cyclo-aspartyl-serine or a salt thereof, content: 1.00 × 10 to 1.60 × 10⁵ ppm;
   (18) cyclo-arginyl-aspartic acid or a salt thereof, content: 0.90 × 10 to 1.50 × 10⁵ ppm;
   (19) cyclo-glycyl-tryptophan or a salt thereof, content: 0.10 × 10 to 0.30 × 10⁵ ppm;
   (20) cyclo-histidyl-phenylalanine or a salt thereof, content: 0.20 × 10 to 0.40 × 10⁵ ppm;
   (21) cyclo-aspartyl-leucine or a salt thereof, content: 0.40 × 10 to 0.80 × 10⁵ ppm;
   (22) cyclo-isoleucyl-histidine or a salt thereof, content: 0.70 × 10 to 1.20 × 10⁵ ppm;
   (23) cyclo-seryl-leucine or a salt thereof, content: 0.70 × 10 to 1.10 × 10⁵ ppm;
   (24) cyclo-isoleucyl-aspartic acid or a salt thereof, content: 0.70 × 10 to 1.10 × 10⁵ ppm;
   (25) cyclo-seryl-cysteine or a salt thereof, content: 0.40 × 10 to 0.70 × 10⁵ ppm;
   (26) cyclo-phenylalanyl-tryptophan or a salt thereof, content: 0.07 × 10 to 0.20 × 10⁵ ppm;
   (27) cyclo-alanyl-leucine or a salt thereof, content: 1.40 × 10 to 2.30 × 10⁵ ppm;
   (28) cyclo-glutaminyl-histidine or a salt thereof, content: 0.40 × 10 to 0.70 × 10⁵ ppm;
   (29) cyclo-arginyl-valine or a salt thereof, content: 0.60 × 10 to 1.00 × 10⁵ ppm;
   (30) cyclo-glutamyl-leucine or a salt thereof, content: 0.70 × 10 to 1.10 × 10⁵ ppm;
   (31) cyclo-leucyl-tryptophan or a salt thereof, content: 0.10 × 10 to 0.30 × 10⁵ ppm;
   (32) cyclo-tryptophanyl-tryptophan or a salt thereof, content: 0.04 × 10 to 0.07 × 10⁵ ppm;
   (33) cyclo-L-alanyl-proline or a salt thereof, content: 0.40 × 10 to 0.70 × 10⁵ ppm;
   (34) cyclo-methionyl-arginine or a salt thereof, content: 0.20 × 10 to 0.50 × 10⁵ ppm;
   (35) cyclo-lysyl-phenylalanine or a salt thereof, content: 1.00 × 10 to 1.60 × 10⁵ ppm;
   (36) cyclo-phenylalanyl-phenylalanine or a salt thereof, content: 0.30 × 10 to 0.60 × 10⁵ ppm;
   (37) cyclo-tryptophanyl-tyrosine or a salt thereof, content: 0.10 × 10 to 0.20 × 10⁵ ppm;
   (38) cyclo-asparaginyl-valine or a salt thereof, content: 0.30 × 10 to 0.50 × 10⁵ ppm;
   (39) cyclo-glutaminyl-isoleucine or a salt thereof, content: 0.50 × 10 to 0.80 × 10⁵ ppm;
   (40) cyclo-alanyl-serine or a salt thereof, content: 0.40 × 10 to 0.80 × 10⁵ ppm;
   (41) cyclo-methionyl-histidine or a salt thereof, content: 0.10 × 10 to 0.30 × 10⁵ ppm;
   (42) cyclo-methionyl-proline or a salt thereof, content: 0.10 × 10 to 0.30 × 10⁵ ppm;
   (43) cyclo-arginyl-leucine or a salt thereof, content: 1.50 × 10 to to 2.30 × 10⁵ ppm;
   (44) cyclo-methionyl-glutamic acid or a salt thereof, content: 0.10 × 10 to 0.30 × 10⁵ ppm;
   (45) cyclo-methionyl-alanine or a salt thereof, content: 0.20 × 10 to 0.50 × 10⁵ ppm;
   (46) cyclo-isoleucyl-glutamic acid or a salt thereof, content: 0.50 × 10 to 0.90 × 10⁵ ppm;
   (47) cyclo-isoleucyl-serine or a salt thereof, content: 0.50 × 10 to 0.90 × 10⁵ ppm;
   (48) cyclo-valyl-serine or a salt thereof, content: 0.50 × 10 to 0.90 × 10⁵ ppm;
   (49) cyclo-methionyl-glycine or a salt thereof, content: 0.40 × 10 to 0.70 × 10⁵ ppm;
   (50) cyclo-valyl-threonine or a salt thereof, content: 0.30 × 10 to 0.60 × 10⁵ ppm;
   (51) cyclo-valyl-aspartic acid or a salt thereof, content: 0.50 × 10 to 0.90 × 10⁵ ppm;
   (52) cyclo-glycyl-proline or a salt thereof, content: 0.30 × 10 to 0.50 × 10⁵ ppm;
   (53) cyclo-leucyl-proline or a salt thereof, content: 0.50 × 10 to 0.90 × 10⁵ ppm;
   (54) cyclo-glutaminyl-glycine or a salt thereof, content: 0.05 × 10 to 0.09 × 10⁵ ppm;
   (55) cyclo-tryptophanyl-lysine or a salt thereof, content: 0.20 × 10 to 0.40 × 10⁵ ppm;
   (56) cyclo-glutaminyl-phenylalanine or a salt thereof, content: 0.30 × 10 to 0.60 × 10⁵ ppm;
   (57) cyclo-lysyl-glycine or a salt thereof, content: 1.00 × 10 to 1.60 × 10⁵ ppm.;
   (58) cyclo-seryl-lysine or a salt thereof, content: 1.60 × 10 to 2.60 × 10⁵ ppm;
   (59) cyclo-valyl-lysine or a salt thereof, content: 0.90 × 10 to 1.50 × 10⁵ ppm;
   (60) cyclo-asparaginyl-lysine or a salt thereof, content: 0.60 × 10 to 1.10 × 10³ ppm;
   (61) cyclo-histidyl-histidine or a salt thereof, content: 0.40 × 10 to 0.70 × 10⁵ ppm;
   (62) cyclo-threonyl-histidine or a salt thereof, content: 0.40 × 10 to 0.70 0 × 10⁵ ppm;
   (63) cyclo-aspartyl-histidine or a salt thereof, content: 0.40 × 10 to 0.70 0 × 10⁵ ppm;
   (64) cyclo-asparaginyl-histidine or a salt thereof, content: 0.10 × 10 to 0.30 × 10⁵ ppm;
   (65) cyclo-arginyl-serine or a salt thereof, content: 0.40 × 10 to 0.70 × 10⁵ ppm;
   (66) cyclo-asparaginyl-methionine or a salt thereof, content: 0.40 × 10 to 0.70 × 10⁵ ppm;
   (67) cyclo-glutaminyl-methionine or a salt thereof, content: 0.10 × 10 to 0.20 × 10⁵ ppm;
   (68) cyclo-tryptophanyl-arginine or a salt thereof, content: 0.10 × 10 to 0.30 × 10⁵ ppm;
   (69) cyclo-asparaginyl-arginine or a salt thereof, content: 0.40 × 10 to 0.70 × 10⁵ ppm;
   (70) cyclo-asparaginyl-proline or a salt thereof, content: 0.05 × 10 to 0.09 9 × 10⁵ ppm; and
   (71) cyclo-arginyl-arginine or a salt thereof, content: 0.40 × 10 to 0.70 × 10⁵ ppm.
3) The composition according to aspect 1) or 2), wherein one or two or more of the following (1) to (10) as the cyclic dipeptide including amino acids as constituent units or salt thereof are each contained in the following content range:
   (1) cyclo-threonyl-tyrosine or a salt thereof, content: 0.40 × 10 to 0.70 × 10⁵ ppm;
   (2) cyclo-valyl-tyrosine or a salt thereof, content: 0.30 × 10 to 0.50 × 10⁵ ppm;
   (3) cyclo-glutaminyl-tyrosine or a salt thereof, content: 0.20 × 10 to 0.40 × 10⁵ ppm;
   (4) cyclo-asparaginyl-isoleucine or a salt thereof, content: 0.40 × 10 to 0.70 × 10⁵ ppm;
   (5) cyclo-arginyl-proline or a salt thereof, content: 0.40 × 10 to 0.70 × 10⁵ ppm;
   (6) cyclo-asparaginyl-glycine or a salt thereof, content: 0.20 × 10 to 0.50 × 10⁵ ppm;
   (7) cyclo-methionyl-valine or a salt thereof, content: 0.40 × 10 to 0.70 × 10⁵ ppm;
   (8) cyclo-glutamyl-cysteine or a salt thereof, content: 0.40 × 10 to 0.70 × 10⁵ ppm;
   (9) cyclo-seryl-glutamic acid or a salt thereof, content: 0.40 × 10 to 0.70 × 10⁵ ppm; and
   (10) cyclo-isoleucyl-lysine or a salt thereof, content: 0.50 × 10 to 0.90 × 10⁵ ppm.
4) A composition containing a cyclic dipeptide including amino acids as constituent units or a salt thereof, wherein the total amount of the cyclic dipeptide including amino acids as constituent units or salt thereof is 0.2 × 10⁻² to 1.0 × 10³ mg/100 mL.
5) The composition according to aspect 4), wherein the cyclic dipeptide including amino acids as constituent units or salt thereof is one or two or more cyclic dipeptide or salts thereof of the following (1) to (71) each contained in an amount satisfying the content range:
   (1) cyclo-threonyl-tyrosine or a salt thereof, content: 0.40 × 10⁻² to 0.70 × 10² mg/100 mL;
   (2) cyclo-valyl-tyrosine or a salt thereof, content: 0.30 × 10⁻² to 0.50 × 10² mg/100 mL;
   (3) cyclo-glutaminyl-tyrosine or a salt thereof, content: 0.20 × 10⁻² to 0.40 × 10² mg/100 mL;
   (4) cyclo-asparaginyl-isoleucine or a salt thereof, content: 0.40 × 10⁻² to 0.70 × 10² mg/100 mL;
   (5) cyclo-arginyl-proline or a salt thereof, content: 0.40 × 10⁻² to 0.70 × 10² mg/100 mL;
   (6) cyclo-asparaginyl-glycine or a salt thereof, content: 0.20 × 10⁻² to 0.50 × 10² mg/100 mL;
   (7) cyclo-methionyl-valine or a salt thereof, content: 0.40 × 10⁻² to 0.70 × 10² mg/100 mL;
   (8) cyclo-glutamyl-cysteine or a salt thereof, content: 0.40 × 10⁻² to 0.70 × 10² mg/100 mL;
   (9) cyclo-seryl-glutamic acid or a salt thereof, content: 0.40 × 10⁻² to 0.70 × 10² mg/100 mL;
   (10) cyclo-isoleucyl-lysine or a salt thereof, content: 0.50 × 10⁻² to 0.90 × 10² mg/100 mL;
   (11) cyclo-glutaminyl-leucine or a salt thereof, content: 0.80 × 10⁻² to 1.30 × 10² mg/100 mL;
   (12) cyclo-isoleucyl-threonine or a salt thereof, content: 0.20 × 10⁻² to 0.50 × 10² mg/100 mL;
   (13) cyclo-threonyl-threonine or a salt thereof, content: 0.30 × 10⁻² to 0.50 × 10² mg/100 mL;
   (14) cyclo-methionyl-threonine or a salt thereof, content: 0.40 × 10⁻² to 0.70 × 10² mg/100 mL;
   (15) cyclo-alanyl-cysteine or a salt thereof, content: 0.40 × 10⁻² to 0.70 × 10² mg/100 mL;
   (16) cyclo-glycyl-cysteine or a salt thereof, content: 0.40 × 10⁻² to 0.70 × 10² mg/100 mL;
   (17) cyclo-aspartyl-serine or a salt thereof, content: 1.00 × 10⁻² to 1.60 × 10² mg/100 mL;
   (18) cyclo-arginyl-aspartic acid or a salt thereof, content: 0.90 × 10⁻² to 1.50 × 10² mg/100 mL;
   (19) cyclo-glycyl-tryptophan or a salt thereof, content: 0.10 × 10⁻² to 0.30 × 10² mg/100 mL;
   (20) cyclo-histidyl-phenylalanine or a salt thereof, content: 0.20 × 10⁻² to 0.40 × 10² mg/100 mL;
   (21) cyclo-aspartyl-leucine or a salt thereof, content: 0.40 × 10⁻² to 0.80 × 10² mg/100 mL;
   (22) cyclo-isoleucyl-histidine or a salt thereof, content: 0.70 × 10⁻² to 1.20 × 10² mg/100 mL;
   (23) cyclo-seryl-leucine or a salt thereof, content: 0.70 × 10⁻² to 1.10 × 10² mg/100 mL;
   (24) cyclo-isoleucyl-aspartic acid or a salt thereof, content: 0.70 × 10⁻² to 1.10 × 10² mg/100 mL;
   (25) cyclo-seryl-cysteine or a salt thereof, content: 0.40 × 10⁻² to 0.70 × 10² mg/100 mL;
   (26) cyclo-phenylalanyl-tryptophan or a salt thereof, content: 0.07 × 10⁻² to 0.20 × 10² mg/100 mL.;
   (27) cyclo-alanyl-leucine or a salt thereof, content: 1.40 × 10⁻² to 2.30 × 10² mg/100 mL;
   (28) cyclo-glutaminyl-histidine or a salt thereof, content: 0.40 × 10⁻² to 0.70 × 10² mg/100 mL;
   (29) cyclo-arginyl-valine or a salt thereof, content: 0.60 × 10⁻² to 1.00 × 10² mg/100 mL;
   (30) cyclo-glutamyl-leucine or a salt thereof, content: 0.70 × 10⁻² to 1.10 × 10² mg/100 mL;
   (31) cyclo-leucyl-tryptophan or a salt thereof, content: 0.10 × 10⁻² to 0.30 × 10² mg/100 mL;
   (32) cyclo-tryptophanyl-tryptophan or a salt thereof, content: 0.04 × 10⁻² to 0.07 × 10² mg/100 mL;
   (33) cyclo-L-alanyl-proline or a salt thereof, content: 0.40 × 10⁻² to 0.70 × 10² mg/100 mL;
   (34) cyclo-methionyl-arginine or a salt thereof, content: 0.20 × 10⁻² to 0.50 × 10² mg/100 mL;
   (35) cyclo-lysyl-phenylalanine or a salt thereof, content: 1.00 × 10⁻² to 1.60 × 10² mg/100 mL;
   (36) cyclo-phenylalanyl-phenylalanine or a salt thereof, content: 0.30 × 10⁻² to 0.60 × 10² mg/100 mL;
   (37) cyclo-tryptophanyl-tyrosine or a salt thereof, content: 0.10 × 10⁻² to 0.20 × 10² mg/100 mL;
   (38) cyclo-asparaginyl-valine or a salt thereof, content: 0.30 × 10⁻² to 0.50 × 10² mg/100 mL;
   (39) cyclo-glutaminyl-isoleucine or a salt thereof, content: 0.50 × 10⁻² to 0.80 × 10² mg/100 mL;
   (40) cyclo-alanyl-serine or a salt thereof, content: 0.40 × 10⁻² to 0.80 × 10² mg/100 mL;
   (41) cyclo-methionyl-histidine or a salt thereof, content: 0.10 × 10⁻² to 0.30 × 10² mg/100 mL;
   (42) cyclo-methionyl-proline or a salt thereof, content: 0.10 × 10⁻² to 0.30 × 10² mg/100 mL;
   (43) cyclo-arginyl-leucine or a salt thereof, content: 1.50 × 10⁻² to 2.30 × 10² mg/100 mL;
   (44) cyclo-methionyl-glutamic acid or a salt thereof, content: 0.10 × 10⁻² to 0.30 × 10² mg/100 mL;
   (45) cyclo-methionyl-alanine or a salt thereof, content: 0.20 × 10⁻² to 0.50 × 10² mg/100 mL;
   (46) cyclo-isoleucyl-glutamic acid or a salt thereof: 0.50 × 10⁻² to 0.90 × 10² mg/100 mL;
   (47) cyclo-isoleucyl-serine or a salt thereof, content: 0.50 × 10⁻² to 0.90 × 10² mg/100 mL;
   (48) cyclo-valyl-serine or a salt thereof, content: 0.50 × 10⁻² to 0.90 × 10² mg/100 mL;
   (49) cyclo-methionyl-glycine or a salt thereof, content: 0.40 × 10⁻² to 0.70 × 10² mg/100 mL;
   (50) cyclo-valyl-threonine or a salt thereof, content: 0.30 × 10⁻² to 0.60 × 10² mg/100 mL;
   (51) cyclo-valyl-aspartic acid or a salt thereof, content: 0.50 × 10⁻² to 0.90 × 10² mg/100 mL;
   (52) cyclo-glycyl-proline or a salt thereof, content: 0.30 × 10⁻² to 0.50 × 10² mg/100 mL;
   (53) cyclo-leucyl-proline or a salt thereof, content: 0.50 × 10⁻² to 0.90 × 10² mg/100 mL;
   (54) cyclo-glutaminyl-glycine or a salt thereof, content: 0.05 × 10⁻² to 0.09 × 10² mg/100 mL;
   (55) cyclo-tryptophanyl-lysine or a salt thereof, content: 0.20 × 10⁻² to 0.40 × 10² mg/100 mL;
   (56) cyclo-glutaminyl-phenylalanine or a salt thereof, content: 0.30 × 10⁻² to 0.60 × 10² mg/100 mL;
   (57) cyclo-lysyl-glycine or a salt thereof, content: 1.00 × 10⁻² to 1.60 × 10² mg/100 mL;
   (58) cyclo-seryl-lysine or a salt thereof, content: 1.60 × 10⁻² to 2.60 × 10² mg/100 mL;
   (59) cyclo-valyl-lysine or a salt thereof, content: 0.90 × 10⁻² to 1.50 × 10² mg/100 mL;
   (60) cyclo-asparaginyl-lysine or a salt thereof, content: 0.60 × 10⁻² to 1.10 × 10² mg/100 mL;
   (61) cyclo-histidyl-histidine or a salt thereof, content: 0.40 × 10⁻² to 0.70 × 10² mg/100 mL;
   (62) cyclo-threonyl-histidine or a salt thereof, content: 0.40 × 10⁻² to 0.70 × 10² mg/100 mL;
   (63) cyclo-aspartyl-histidine or a salt thereof, content: 0.40 × 10⁻² to 0.70 × 10² mg/100 mL;
   (64) cyclo-asparaginyl-histidine or a salt thereof, content: 0.10 × 10⁻² to 0.30 × 10² mg/100 mL;
   (65) cyclo-arginyl-serine or a salt thereof, content: 0.40 × 10⁻² to 0.70 × 10² mg/100 mL;
   (66) cyclo-asparaginyl-methionine or a salt thereof, content: 0.40 × 10⁻² to 0.70 × 10² mg/100 mL;
   (67) cyclo-glutaminyl-methionlne or a salt thereof, content: 0.10 × 10⁻² to 0.20 × 10² mg/100 mL;
   (68) cyclo-tryptophanyl-arginine or a salt thereof, content: 0.10 × 10⁻² to 0.30 × 10² mg/100 mL;
   (69) cyclo-asparaginyl-arginine or a salt thereof, content: 0.40 × 10⁻² to 0.70 × 10² mg/100 mL;
   (70) cyclo-asparaginyl-proline or a salt thereof, content: 0.05 × 10⁻² to 0.09 × 10² mg/100 mL; and
   (71) cyclo-arginyl-arginine or a salt thereof, content: 0.40 × 10⁻² to 0.70 × 10² mg/100 mL.
6) The composition according to aspect 4) or 5), wherein one or two or more of the following (1) to (10) as the cyclic dipeptide including amino acids as constituent units or salt thereof are each contained in the following content range:
   (1) cyclo-threonyl-tyrosine or a salt thereof, content: 0.40 × 10⁻² to 0.70 × 10² mg/100 mL;
   (2) cyclo-valyl-tyrosine or a salt thereof, content: 0.30 × 10⁻² to 0.50 × 10² mg/100 mL;
   (3) cyclo-glutaminyl-tyrosine or a salt thereof, content: 0.20 × 10⁻² to 0.40 × 10² mg/100 mL;
   (4) cyclo-asparaginyl-isoleucine or a salt thereof, content: 0.40 × 10⁻² to 0.70 × 10² mg/100 mL;
   (5) cyclo-arginyl-proline or a salt thereof, content: 0.40 × 10⁻² to 0.70 × 10² mg/100 mL;
   (6) cyclo-asparaginyl-glycine or a salt thereof, content: 0.20 × 10⁻² to 0.50 × 10² mg/100 mL;
   (7) cyclo-methionyl-valine or a salt thereof, content: 0.40 × 10⁻² to 0.70 × 10² mg/100 mL;
   (8) cyclo-glutamyl-cysteine or a salt thereof, content: 0.40 × 10⁻² to 0.70 × 10² mg/100 mL;
   (9) cyclo-seryl-glutamic acid or a salt thereof, content: 0.40 × 10⁻² to 0.70 × 10² mg/100 mL; and
   (10) cyclo-isoleucyl-lysine or a salt thereof, content: 0.50 × 10⁻² to 0.90 × 10² mg/100 mL.
7) A composition containing a cyclic dipeptide including amino acids as constituent units or a salt thereof, wherein the total amount of the cyclic dipeptide including amino acids as constituent units or salt thereof is 0.20 × 10⁻³ to 100% by weight.
8) The composition according to aspect 7), wherein the cyclic dipeptide including amino acids as constituent units or salt thereof is one or two or more cyclic dipeptides or salts thereof of the following (1) to (71) each contained in an amount satisfying the content range:
   (1) cyclo-threonyl-tyrosine or a salt thereof, content: 0.40 × 10⁻³ to 0.70 × 10% by weight;
   (2) cyclo-valyl-tyrosine or a salt thereof, content: 0.30 × 10⁻³ to 0.50 × 10% by weight;
   (3) cyclo-glutaminyl-tyrosine or a salt thereof, content: 0.20 × 10⁻³ to 0.40 × 10% by weight;
   (4) cyclo-asparaginyl-isoleucine or a salt thereof, content: 0.40 × 10⁻³ to 0.70 × 10% by weight;
   (5) cyclo-arginyl-proline or a salt thereof, content: 0.40 × 10⁻³ to 0.70 × 10% by weight;
   (6) cyclo-asparaginyl-glycine or a salt thereof, content: 0.20 × 10⁻³ to 0.50 × 10% by weight;
   (7) cyclo-methionyl-valine or a salt thereof, content: 0.40 × 10⁻³ to 0.70 × 10% by weight;
   (8) cyclo-glutamyl-cysteine or a salt thereof, content: 0.40 × 10⁻³ to 0.70 × 10% by weight;
   (9) cyclo-seryl-glutamic acid or a salt thereof, content: 0.40 × 10⁻³ to 0.70 × 10% by weight;
   (10) cyclo-isoleucyl-lysine or a salt thereof, content: 0.50 × 10⁻³ to 0.90 × 10% by weight;
   (11) cyclo-glutaminyl-leucine or a salt thereof, content: 0.80 × 10⁻³ to 1.30 × 10% by weight;
   (12) cyclo-isoleucyl-threonine or a salt thereof, content: 0.20 × 10⁻³ to 0.50 × 10% by weight;
   (13) cyclo-threonyl-threonine or a salt thereof, content: 0.30 × 10⁻³ to 0.50 × 10% by weight;
   (14) cyclo-methionyl-threonine or a salt thereof, content: 0.40 × 10⁻³ to 0.70 × 10% by weight;
   (15) cyclo-alanyl-cysteine or a salt thereof, content: 0.40 × 10⁻³ to 0.70 × 10% by weight;
   (16) cyclo-glycyl-cysteine or a salt thereof, content: 0.40 × 10⁻³ to 0.70 × 10% by weight;
   (17) cyclo-aspartyl-serine or a salt thereof, content: 1.00 × 10⁻³ to 1.60 × 10% by weight;
   (18) cyclo-arginyl-aspartic acid or a salt thereof, content: 0.90 × 10⁻³ to 1.50 × 10% by weight;
   (19) cyclo-glycyl-tryptophan or a salt thereof, content: 0.10 × 10⁻³ to 0.30 × 10% by weight;
   (20) cyclo-histidyl-phenylalanine or a salt thereof, content: 0.20 × 10⁻³ to 0.40 × 10% by weight;
   (21) cyclo-aspartyl-leucine or a salt thereof, content: 0.40 × 10⁻³ to 0.80 × 10% by weight;
   (22) cyclo-isoleucyl-histidine or a salt thereof, content: 0.70 × 10⁻³ to 1.20 × 10% by weight;
   (23) cyclo-seryl-leucine or a salt thereof, content: 0.70 × 10⁻³ to 1.10 × 10% by weight;
   (24) cyclo-isoleucyl-aspartic acid or a salt thereof, content: 0.70 × 10⁻³ to 1.10 × 10% by weight;
   (25) cyclo-seryl-cysteine or a salt thereof, content: 0.40 × 10⁻³ to 0.70 × 10% by weight;
   (26) cyclo-phenylalanyl-tryptophan or a salt thereof, content: 0.07 × 10⁻³ to 0.20 × 10% by weight;
   (27) cyclo-alanyl-leucine or a salt thereof, content: 1.40 × 10⁻³ to 2.30 × 10% by weight;
   (28) cyclo-glutaminyl-histidine or a salt thereof, content: 0.40 × 10⁻³ to 0.70 × 10% by weight;
   (29) cyclo-arginyl-valine or a salt thereof, content: 0.60 × 10⁻³ to 1.00 × 10% by weight;
   (30) cyclo-glutamyl-leucine or a salt thereof, content: 0.70 × to 1.10 × 10% by weight;
   (31) cyclo-leucyl-tryptophan or a salt thereof, content: 0.10 × 10⁻³ to 0.30 × 10% by weight;
   (32) cyclo-tryptophanyl-tryptophan or a salt thereof, content: 0.04 × 10⁻³ to 0.07 × 10% by weight;
   (33) cyclo-L-alanyl-proline or a salt thereof, content: 0.40 × 10⁻³ to 0.70 × 10% by weight;
   (34) cyclo-methionyl-arginine or a salt thereof, content: 0.20 × 10⁻³ to 0.50 × 10% by weight;
   (35) cyclo-lysyl-phenylalanine or a salt thereof, content: 1.00 × 10⁻³ to 1.60 × 10% by weight;
   (36) cyclo-phenylalanyl-phenylalanine or a salt thereof, content: 0.30 × 10⁻³ to 0.60 × 10% by weight;
   (37) cyclo-tryptophanyl-tyrosine or a salt thereof, content: 0.10 × 10⁻³ to 0.20 × 10% by weight;
   (38) cyclo-asparaginyl-valine or a salt thereof, content: 0.30 × 10⁻³ to 0.50 × 10% by weight;
   (39) cyclo-glutaminyl-isoleucine or a salt thereof, content: 0.50 × 10⁻³ to 0.80 × 10% by weight;
   (40) cyclo-alanyl-serine or a salt thereof, content: 0.40 × 10⁻³ to 0.80 × 10% by weight;
   (41) cyclo-methionyl-histidine or a salt thereof, content: 0.10 × 10⁻³ to 0.30 × 10% by weight;
   (42) cyclo-methionyl-proline or a salt thereof, content: 0.10 × 10⁻³ to 0.30 × 10% by weight;
   (43) cyclo-arginyl-leucine or a salt thereof, content: 1.50 × 10⁻³ to 2.30 × 10% by weight;
   (44) cyclo-methionyl-glutamic acid or a salt thereof, content: 0.10 × 10⁻³ to 0.30 × 10% by weight;
   (45) cyclo-methionyl-alanine or a salt thereof, content: 0.20 × 10⁻³ to 0.50 × 10% by weight;
   (46) cyclo-isoleucyl-glutamic acid or a salt thereof, content: 0.50 × 10⁻³ to 0.90 × 10% by weight;
   (47) cyclo-isoleucyl-serine or a salt thereof, content: 0.50 × 10⁻³ to 0.90 × 10% by weight;
   (48) cyclo-valyl-serine or a salt thereof, content: 0.50 × 10⁻³ to 0.90 × 10% by weight;
   (49) cyclo-methionyl-glycine or a salt thereof, content: 0.40 × 10⁻³ to 0.70 × 10% by weight;
   (50) cyclo-valyl-threonine or a salt thereof, content: 0.30 × 10⁻³ to 0.60 × 10% by weight;
   (51) cyclo-valyl-aspartic acid or a salt thereof, content: 0.50 × 10⁻³ to 0.90 × 10% by weight;
   (52) cyclo-glycyl-proline or a salt thereof, content: 0.30 × 10⁻³ to 0.50 × 10% by weight;
   (53) cyclo-leucyl-proline or a salt thereof, content: 0.50 × 10⁻³ to 0.90 × 10% by weight;
   (54) cyclo-glutaminyl-glycine or a salt thereof, content: 0.05 × 10⁻³ to 0.09 × 10% by weight;
   (55) cyclo-tryptophanyl-lysine or a salt thereof, content: 0.20 × 10⁻³ to 0.40 × 10% by weight;
   (56) cyclo-glutaminyl-phenylalanine or a salt thereof, content: 0.30 × 10⁻³ to 0.60 × 10% by weight;
   (57) cyclo-lysyl-glycine or a salt thereof, content: 1.00 × 10⁻³ to 1.60 × 10% by weight;
   (58) cyclo-seryl-lysine or a salt thereof, content: 1.60 × 10⁻³ to 2.60 × 10% by weight;
   (59) cyclo-valyl-lysine or a salt thereof, content: 0.90 × 10⁻³ to 1.50 × 10% by weight;
   (60) cyclo-asparaginyl-lysine or a salt thereof, content: 0.60 × 10⁻³ to 1.10 × 10% by weight;
   (61) cyclo-histidyl-histidine or a salt thereof, content: 0.40 × 10⁻³ to 0.70 × 10% by weight;
   (62) cyclo-threonyl-histidine or a salt thereof, content: 0.40 × 10⁻³ to 0.70 × 10% by weight;
   (63) cyclo-aspartyl-histidine or a salt thereof, content: 0.40 × 10⁻³ to 0.70 × 10% by weight;
   (64) cyclo-asparaginyl-histidine or a salt thereof, content: 0.10 × 10⁻³ to 0.30 × 10% by weight;
   (65) cyclo-arginyl-serine or a salt thereof, content: 0.40 × 10⁻³ to 0.70 × 10% by weight;
   (66) cyclo-asparaginyl-methionine or a salt thereof, content: 0.40 × 10⁻³ to 0.70 × 10% by weight;
   (67) cyclo-glutaminyl-methionine or a salt thereof, content: 0.10 × 10⁻³ to 0.20 × 10% by weight;
   (68) cyclo-tryptophanyl-arginine or a salt thereof, content: 0.10 × 10⁻³ to 0.30 × 10% by weight;
   (69) cyclo-asparaginyl-arginine or a salt thereof, content: 0.40 × 10⁻³ to 0.70 × 10% by weight;
   (70) cyclo-asparaginyl-proline or a salt thereof, content: 0.05 × 10⁻³ to 0.09 × 10% by weight; and
   (71) cyclo-arginyl-arginine or a salt thereof, content: 0.40 × 10⁻³ 0.70 × 10% by weight.
9) The composition according to aspect 7) or 8), wherein one or two or more of the following (1) to (10) as the cyclic dipeptide including amino acids as constituent units or salt thereof are each contained in the following content range:
   (1) cyclo-threonyl-tyrosine or a salt thereof, content: 0.40 × 10⁻³ to 0.70 × 10% by weight;
   (2) cyclo-valyl-tyrosine or a salt thereof, content: 0.30 × 10⁻³ to 0.50 × 10% by weight;
   (3) cyclo-glutaminyl-tyrosine or a salt thereof, content: 0.20 × 10⁻³ to 0.40 × 10% by weight;
   (4) cyclo-asparaginyl-isoleucine or a salt thereof, content: 0.40 × 10⁻³ to 0.70 × 10% by weight;
   (5) cyclo-arginyl-proline or a salt thereof, content: 0.40 × 10⁻³ to 0.70 × 10% by weight;
   (6) cyclo-asparaginyl-glycine or a salt thereof, content: 0.20 × 10⁻³ to 0.50 × 10% by weight;
   (7) cyclo-methionyl-valine or a salt thereof, content: 0.40 × 10⁻³ to 0.70 × 10% by weight;
   (8) cyclo-glutamyl-cysteine or a salt thereof, content: 0.40 × 10⁻³ to 0.70 × 10% by weight;
   (9) cyclo-seryl-glutamic acid or a salt thereof, content: 0.40 × 10⁻³ to 0.70 × 10% by weight; and
   (10) cyclo-isoleucyl-lysine or a salt thereof, content: 0.50 × 10⁻³ to 0.90 × 10% by weight.
10) The composition according to any one of aspects 1) to 9), wherein the cyclic dipeptide including amino acids as constituent units is derived from soybean.
11) The composition according to any one of aspects 1) to 9), wherein the cyclic dipeptide including amino acids as constituent units is derived from tea.

### ADVANTAGEOUS EFFECTS OF INVENTION

The composition with a high content of a cyclic dipeptide including amino acids as constituent units according to the present invention is safe and can be ingested for a long period, and has excellent physiological actions such as a glucose metabolism-ameliorating action.

### BRIEF DESCRIPTION OF DRAWINGS

Fig. 1 shows the plasma active GLP-1 level after administration of a heat-treated soybean peptide.
Fig. 2 shows the results of a glucose-loading test after administration of a heat-treated soybean peptide and a soybean peptide.
Fig. 3 shows the results of a glucose-loading test after administration of a heat-treated tea peptide.

### DESCRIPTION OF EMBODIMENTS

One aspect of the present invention is a composition containing a cyclic dipeptide including amino acids as constituent units or a salt thereof, in which the total amount of the cyclic dipeptide including amino acids as constituent units or salt thereof is 0.2 × 10 to 1.0 × 10⁶ ppm. Further, one aspect of the present invention is a composition containing a cyclic dipeptide including amino acids as constituent units or a salt thereof, in which the total amount of the cyclic dipeptide including amino acids as constituent units or salt thereof is 0.2 × 10⁻² to 1.0 × 10³ mg/100 mL. Furthermore, one aspect of the present invention is a composition containing a cyclic dipeptide including amino acids as constituent units or a salt thereof, in which the total amount of the cyclic dipeptide including amino acids as constituent units or salt thereof is 0.20 × 10⁻³ to 100% by weight.

More preferably, one aspect of the present invention is a composition containing a cyclic dipeptide including amino acids as constituent units or a salt thereof, in which the total amount of the cyclic dipeptide including amino acids as constituent units or salt thereof is 2 × 10² to 1.0 × 10⁵ ppm. Further, one aspect of the present invention is a composition containing a cyclic dipeptide including amino acids as constituent units or a salt thereof, in which the total amount of the cyclic dipeptide including amino acids as constituent units or salt thereof is 0.2 × 10⁻¹ to 1.0 × 10² mg/100 mL, and more preferably 0.2 × 10 to 1.0 × 10³ mg/100 mL. Furthermore, one aspect of the present invention is a composition containing a cyclic dipeptide including amino acids as constituent units or a salt thereof, in which the total amount of the cyclic dipeptide including amino acids as constituent units or salt thereof is 0.20 × 10⁻² to 10% by weight.

Here, the total amount of the cyclic dipeptide including amino acids as constituent units or salt thereof refers to the total amount of cyclic dipeptides of group A consisting of cyclo-threonyl-tyrosine [Cyclo(Thr-Tyr)], cyclo-valyl-tyrosine [Cyclo(Val-Tyr)], cyclo-glutaminyl-tyrosine [Cyclo(Gln-Tyr)], cyclo-asparaginyl-isoleucine [Cyclo(Asn-Ile)], cyclo-arginyl-proline [Cyclo(Arg-Pro)], cyclo-asparaginyl-glycine [Cyclo(Asn-Gly)], cyclo-methionyl-valine [Cyclo(Met-Val)], cyclo-glutamyl-cysteine [Cyclo(Glu-Cys)], cyclo-seryl-glutamic acid [Cyclo(Ser-Glu)], cyclo-isoleucyl-lysine [Cyclo(Ile-Lys)], cyclo-glutaminyl-leucine [Cyclo(Gln-Leu)], cyclo-isoleucyl-threonine [Cyclo(Ile-Thr)], cyclo-threonyl-threonine [Cyclo(Thr-Thr)], cyclo-methionyl-threonine [Cyclo(Met-Thr)], cyclo-alanyl-cysteine [Cyclo(Ala-Cys)], cyclo-glycyl-cysteine [Cyclo(Gly-Cys)], cyclo-aspartyl-serine [Cyclo(Asp-Ser)], cyclo-arginyl-aspartic acid [Cyclo(Arg-Asp)], cyclo-glycyl-tryptophan [Cyclo(Gly-Trp)], cyclo-histidyl-phenylalanine [Cyclo(His-Phe)], cyclo-aspartyl-leucine [Cyclo(Asp-Leu)], cyclo-isoleucyl-histidine [Cyclo(Ile-His)], cyclo-seryl-leucine [Cyclo(Ser-Leu)], cyclo-isoleucyl-aspartic acid [Cyclo(Ile-Asp)], cyclo-seryl-cysteine [Cyclo(Ser-Cys)], cyclo-phenylalanyl-tryptophan [Cyclo(Phe-Trp)], cyclo-alanyl-leucine [Cyclo(Ala-Leu)], cyclo-glutaminyl-histidine [Cyclo(Gln-His)], cyclo-arginyl-valine [Cyclo(Arg-Val)], cyclo-glutamyl-leucine [Cyclo(Glu-Leu)], cyclo-leucyl-tryptophan [Cyclo(Leu-Trp)], cyclo-tryptophanyl-tryptophan [Cyclo(Trp-Trp)], cyclo-L-alanyl-proline [Cyclo(L-Ala-Pro)], cyclo-methionyl-arginine [Cyclo(Met-Arg)], cyclo-lysyl-phenylalanine [Cyclo(Lys-Phe)], cyclo-phenylalanyl-phenylalanine [Cyclo(Phe-Phe)], cyclo-tryptophanyl-tyrosine [Cyclo(Trp-Tyr)], cyclo-asparaginyl-valine [Cyclo(Asn-Val)], cyclo-glutaminyl-isoleucine [Cyclo(Gln-Ile)], cyclo-alanyl-serine [Cyclo(Ala-Ser)], cyclo-methionyl-histidine [Cyclo(Met-His)], cyclo-methionyl-proline [Cyclo(Met-Pro)], cyclo-arginyl-leucine [Cyclo(Arg-Leu)], cyclo-methionyl-glutamic acid [Cyclo(Met-Glu)], cyclo-methionyl-alanine [Cyclo(Met-Ala)], cyclo-isoleucyl-glutamic acid [Cyclo(Ile-Glu)], cyclo-isoleucyl-serine [Cyclo(Ile-Ser)], cyclo-valyl-serine [Cyclo(Val-Ser)], cyclo-methionyl-glycine [Cyclo(Met-Gly)], cyclo-valyl-threonine [Cyclo(Val-Thr)], cyclo-valyl-aspartic acid [Cyclo(Val-Asp)], cyclo-glycyl-proline [Cyclo(Gly-Pro)], cyclo-leucyl-proline [Cyclo(Leu-Pro)], cyclo-glutaminyl-glycine [Cyclo(Gln-Gly)], cyclo-tryptophanyl-lysine [Cyclo(Trp-Lys)], cyclo-glutaminyl-phenylalanine [Cyclo(Gln-Phe)], cyclo-lysyl-glycine [Cyclo(Lys-Gly)], cyclo-seryl-lysine [Cyclo(Ser-Lys)], cyclo-valyl-lysine [Cyclo(Val-Lys)], cyclo-asparaginyl-lysine [Cyclo(Asn-Lys)], cyclo-histidyl-histidine [Cyclo(His-His)], cyclo-threonyl-histidine [Cyclo(Thr-His)], cyclo-aspartyl-histidine [Cyclo(Asp-His)], cyclo-asparaginyl-histidine [Cyclo(Asn-His)], cyclo-arginyl-serine [Cyclo(Arg-Ser)], cyclo-asparaginyl-methionine [Cyclo(Asn-Met)], cyclo-glutaminyl-methionine [Cyclo(Gln-Met)], cyclo-tryptophanyl-arginine [Cyclo(Trp-Arg)], cyclo-asparaginyl-arginine [Cyclo(Asn-Arg)], cyclo-asparaginyl-proline [Cyclo(Asn-Pro)], and cyclo-arginyl-arginine [Cyclo(Arg-Arg)], or salts thereof.

The composition with a high content of a cyclic dipeptide including amino acids as constituent units according to the present invention contains one or two or more cyclic dipeptides selected from group A or salts thereof, and is characterized in that the cyclic dipeptide or salt thereof is contained in a specific amount. Throughout the specification, the order of amino acids in a cyclic dipeptide may be inverse as long as the constitution is unchanged, and for example, Cyclo(Trp-Tyr) and Cyclo(Tyr-Trp) represent an identical cyclic dipeptide.

Let two amino acids constituting the cyclic dipeptide according to the present invention be amino acid A and amino acid B. The cyclic dipeptide according to the present invention has a structure in which the carboxy group of amino acid A and the amino group of amino acid B have undergone dehydration condensation and the amino group of amino acid A and the carboxy group of amino acid B have undergone dehydration condensation. At least one of the constituent amino acids is preferably a basic amino acid, and more preferably arginine, but is not limited thereto.

The composition with a high content of a cyclic dipeptide according to the present invention is only required to contain one or two or more cyclic dipeptides of the above-mentioned 71 cyclic dipeptides in group A or salts thereof each in a specific amount. Throughout the specification, cyclic dipeptides or salts thereof are occasionally referred to as cyclic dipeptides in a collective and simple manner.

The specific amounts are as follows. The following amounts are applied to Embodiments 1 to 3. Although the specific amounts are represented in ppm in the following, it is obvious to those skilled in the art that ppm can be appropriately converted to mg/100 mL or % by weight.
(1) cyclo-threonyl-tyrosine or a salt thereof, content: 0.40 × 10 to 0.70 × 10⁵ ppm, preferably 0.40 × 10² to 0.70 × 10⁴ ppm.
(2) cyclo-valyl-tyrosine or a salt thereof, content: 0.30 × 10 to 0.50 × 10⁵ ppm, preferably (0.30 × 10² to 0.50 × 10⁴ ppm.
(3) cyclo-glutaminyl-tyrosine or a salt thereof, content: 0.20 × 10 to 0.40 × 10⁵ ppm, preferably 0.20 × 10² to 0.40 × 10⁴ ppm.
(4) cyclo-asparaginyl-isoleucine or a salt thereof, content: 0.40 × 10 to 0.70 × 10⁵ ppm, preferably 0.40 × 10² to 0.70 × 10⁴ ppm.
(5) cyclo-arginyl-proline or a salt thereof, content: 0.40 × 10 to 0.70 × 10⁵ ppm, preferably 0.40 × 10² to 0.70 × 10⁴ ppm.
(6) cyclo-asparaginyl-glycine or a salt thereof, content: 0.20 × 10 to 0.50 × 10⁵ ppm, preferably 0.20 × 10² to 0.50 × 10⁴ ppm.
(7) cyclo-methionyl-valine or a salt thereof, content: 0.40 × 10 to 0.70 × 10⁵ ppm, preferably 0.40 × 10² to 0.70 × 10⁴ ppm.
(8) cyclo-glutamyl-cysteine or a salt thereof, content: 0.40 × 10 to 0.70 × 10⁵ ppm, preferably 0.40 × 10² to 0.70 × 10⁴ ppm.
(9) cyclo-seryl-glutamic acid or a salt thereof, content: 0.40 × 10 to 0.70 × 10⁵ ppm, preferably 0.40 × 10² to 0.70 × 10⁴ ppm.
(10) cyclo-isoleucyl-lysine or a salt thereof, content: 0.50 × 10 to 0.90 × 10⁵ ppm, preferably 0.50 × 10² to 0.90 × 10⁴ ppm.
(11) cyclo-glutaminyl-leucine or a salt thereof, content: 0.80 × 10 to 1.30 × 10⁵ ppm, preferably 0.80 x 10² to 1.30 × 10⁴ ppm.
(12) cyclo-isoleucyl-threonine or a salt thereof, content: 0.20 × 10 to 0.50 × 10⁵ ppm, preferably 0.20 × 10² to 0.50 × 10⁴ ppm.
(13) cyclo-threonyl-threonine or a salt thereof, content: 0.30 × 10 to 0.50 × 10⁵ ppm, preferably 0.30 × 10² to 0.50 × 10⁴ ppm.
(14) cyclo-methionyl-threonine or a salt thereof, content: 0.40 × 10 to 0.70 × 10⁵ ppm, preferably 0.40 × 10² to 0.70 × 10⁴ ppm.
(15) cyclo-alanyl-cysteine or a salt thereof, content: 0.40 × 10 to 0.70 × 10⁵ ppm, preferably 0.40 × 10² to 0.70 × 10⁴ ppm.
(16) cyclo-glycyl-cysteine or a salt thereof, content: 0.40 × 10 to 0.70 × 10⁵ ppm, preferably 0.40 × 10² to 0.70 × 10⁴ ppm.
(17) cyclo-aspartyl-serine or a salt thereof, content: 1.00 × 10 to 1.60 × 10⁵ ppm, preferably 1.00 × 10² to 1.60 × 10⁴ ppm.
(18) cyclo-arginyl-aspartic acid or a salt thereof, content: 0.90 × 10 to 1.50 × 10⁵ ppm, preferably 0.90 × 10² to 1.50 × 10⁴ ppm.
(19) cyclo-glycyl-tryptophan or a salt thereof, content: 0.10 × 10 to 0.30 × 10⁵ ppm, preferably 0.10 × 10² to 0.30 × 10⁴ ppm.
(20) cyclo-histidyl-phenylalanine or a salt thereof, content: 0.20 × 10 to 0.40 × 10⁵ ppm, preferably 0.20 × 10² to 0.40 × 10⁴ ppm.
(21) cyclo-aspartyl-leucine for a salt thereof, content: 0.40 × 10 to 0.80 × 10⁵ ppm, preferably 0.40 × 10² to 0.80 × 10⁴ ppm.
(22) cyclo-isoleucyl-histidine or a salt thereof, content: 0.70 × 10 to 1.20 × 10⁵ ppm, preferably 0.70 × 10² to 1.20 × 10⁴ ppm.
(23) cyclo-seryl-leucine or a salt thereof, content: 0.70 × 10 to 1.10 × 10⁵ ppm, preferably 0.70 × 10² to 1.10 × 10⁴ ppm.
(24) cyclo-isoleucyl-aspartic acid or a salt thereof, content: 0.70 × 10 to 1.10 × 10⁵ ppm, preferably 0.70 × 10² to 1.10 × 10⁴ ppm.
(25) cyclo-seryl-cysteine or a salt thereof, content: 0.40 × 10 to 0.70 × 10⁵ ppm, preferably 0.40 × 10² to 0.70 × 10⁴ ppm.
(26) cyclo-phenylalanyl-tryptophan or a salt thereof, content: 0.07 × 10 to 0.20 × 10⁵ ppm, preferably 0.07 × 10² to 0.20 × 10⁴ ppm.
(27) cyclo-alanyl-leucine or a salt thereof, content: 1.40 × 10 to 2.30 × 10⁵ ppm, preferably 1.40 × 10² to 2.30 × 10⁴ ppm.
(28) cyclo-glutaminyl-histidine or a salt thereof, content: 0.40 × 10 to 0.70 × 10⁵ ppm, preferably 0.40 × 10² to 0.70 × 10⁴ ppm.
(29) cyclo-arginyl-valine or a salt thereof, content: 0.60 × 10 to 1.00 × 10⁵ ppm, preferably 0.60 × 10² to 1.00 × 10⁴ ppm.
(30) cyclo-glutamyl-leucine or a salt thereof, content: 0.70 × 10 to 1.10 × 10⁵ ppm, preferably 0.70 × 10² to 1.10 × 10⁴ ppm.
(31) cyclo-leucyl-tryptophan or a salt thereof, content: 0.10 × 10 to 0.30 × 10⁵ ppm, preferably 0.10 × 10² to 0.30 × 10⁴ ppm.
(32) cyclo-tryptophanyl-tryptophan or a salt thereof, content: 0.04 × 10 to 0.07 × 10⁵ ppm, preferably 0.04 × 10² to 0.07 × 10⁴ ppm.
(33) cyclo-L-alanyl-proline or a salt thereof, content: 0.40 × 10 to 0.70 × 10⁵ ppm, preferably 0.40 × 10² to 0.70 × 10⁴ ppm.
(34) cyclo-methionyl-arginine or a salt thereof, content: 0.20 × 10 to 0.50 × 10⁵ ppm, preferably 0.20 × 10² to 0.50 × 10⁴ ppm.
(35) cyclo-lysyl-phenylalanine or a salt thereof, content: 1.00 × 10 to 1.60 × 10⁵ ppm, preferably 1.00 × 10² to 1.60 × 10⁴ ppm.
(36) cyclo-phenylalanyl-phenylalanine or a salt thereof, content: 0.30 × 10 to 0.60 × 10⁵ ppm, preferably 0.30 × 10² to 0.60 × 10⁴ ppm.
(37) cyclo-tryptophanyl-tyrosine or a salt thereof, content: 0.10 × 10 to 0.20 × 10⁵ ppm, preferably 0.10 × 10² to 0.20 × 10⁴ ppm.
(38) cyclo-asparaginyl-valine or a salt thereof, content: 0.30 × 10 to 0.50 × 10⁵ ppm, preferably 0.30 × 10² to 0.50 × 10⁴ ppm.
(39) cyclo-glutaminyl-isoleucine or a salt thereof, content: 0.50 × 10 to 0.80 × 10⁵ ppm, preferably 0.50 × 10² to 0.80 × 10⁴ ppm.
(40) cyclo-alanyl-serine or a salt thereof, content: 0.40 × 10 to 0.80 × 10⁵ ppm, preferably 0.40 × 10² to 0.80 × 10⁴ ppm.
(41) cyclo-methionyl-histidine or a salt thereof, content: 0.10 × 10 to 0.30 × 10⁵ ppm, preferably 0.10 × 10² to 0.30 × 10⁴ ppm.
(42) cyclo-methionyl-proline or a salt thereof, content: 0.10 × 10 to 0.30 × 10⁵ ppm, preferably 0.10 × 10² to 0.30 × 10⁴ ppm.
(43) cyclo-arginyl-leucine or a salt thereof, content: 1.50 × 10 to 2.30 × 10⁵ ppm, preferably 1.50 × 10² to 2.30 × 10⁴ ppm.
(44) cyclo-methionyl-glutamic acid or a salt thereof, content: 0.10 × 10 to 0.30 × 10⁵ ppm, preferably 0.10 × 10² to 0.30 × 10⁴ ppm.
(45) cyclo-methionyl-alanine or a salt thereof, content: 0.20 × 10 to 0.50 × 10⁵ ppm, preferably 0.20 × 10² to 0.50 × 10⁴ ppm.
(46) cyclo-isoleucyl-glutamic acid or a salt thereof: 0.50 × 10 to 0.90 × 10⁵ ppm, preferably 0.50 × 10² to 0.90 × 10⁴ ppm.
(47) cyclo-isoleucyl-serine or a salt thereof, content: 0.50 × 10 to 0.90 × 10⁵ ppm, preferably 0.50 × 10² to 0.90 × 10⁴ ppm.
(48) cyclo-valyl-serine or a salt thereof, content: 0.50 × 10 to 0.90 × 10⁵ ppm, preferably 0.50 × 10² to 0.90 × 10⁴ ppm.
(49) cyclo-methionyl-glycine or a salt thereof, content: 0.40 × 10 to 0.70 × 10⁵ ppm, preferably 0.40 × 10² to 0.70 × 10⁴ ppm.
(50) cyclo-valyl-threonine or a salt thereof, content: 0.30 × 10 to 0.60 × 10⁵ ppm, preferably 0.30 × 10² to 0.60 × 10⁴ ppm.
(51) cyclo-valyl-aspartic acid or a salt thereof, content: 0.50 × 10 to 0.90 × 10⁵ ppm, preferably 0.50 × 10² to 0.90 × 10⁴ ppm.
(52) cyclo-glycyl-proline or a salt thereof, content: 0.30 × 10 to 0.50 × 10⁵ ppm, preferably 0.30 × 10² to 0.50 × 10⁴ ppm.
(53) cyclo-leucyl-proline or a salt thereof, content: 0.50 × 10 to 0.90 × 10⁵ ppm, preferably 0.50 × 10² to 0.90 × 10⁴ ppm.
(54) cyclo-glutaminyl-glycine or a salt thereof, content: 0.05 × 10 to 0.09 × 10⁵ ppm, preferably 0.05 × 10² to 0.09 × 10⁴ ppm.
(55) cyclo-tryptophanyl-lysine or a salt thereof, content: 0.20 × 10 to 0.40 × 10⁵ ppm, preferably 0.20 × 10² to 0.40 × 10⁴ ppm.
(56) cyclo-glutaminyl-phenylalanine or a salt thereof, content: 0.30 × 10 to 0.60 × 10⁵ ppm, preferably 0.30 × 10² to 0.60 × 10⁴ ppm.
(57) cyclo-lysyl-glycine or a salt thereof, content: 1.00 × 10 to 1.60 × 10⁵ ppm, preferably 1.00 × 10² to 1.60 × 10⁴ ppm.
(58) cyclo-seryl-lysine or a salt thereof, content: 1.60 × 10 to 2.60 × 10⁵ ppm, preferably 1.60 × 10² to 2.60 × 10⁴ ppm.
(59) cyclo-valyl-lysine or a salt thereof, content: 0.90 × 10 to 1.50 × 10⁵ ppm, preferably 0.90 × 10² to 1.50 × 10⁴ ppm.
(60) cyclo-asparaginyl-lysine or a salt thereof, content: 0.60 × 10 to 1.10 × 10⁵ ppm, preferably 0.60 × 10² to 1.10 × 10⁴ ppm.
(61) cyclo-histidyl-histidine or a salt thereof, content: 0.40 × 10 to 0.70 × 10⁵ ppm, preferably 0.40 × 10² to 0.70 × 10⁴ ppm.
(62) cyclo-threonyl-histidine or a salt thereof, content: 0.40 × 10 to 0.70 × 10⁵ ppm, preferably 0.40 × 10² to 0.70 × 10⁴ ppm.
(63) cyclo-aspartyl-histidine or a salt thereof, content: 0.40 × 10 to 0.70 × 10⁵ ppm, preferably 0.40 × 10² to 0.70 × 10⁴ ppm.
(64) cyclo-asparaginyl-histidine or a salt thereof, content: 0.10 × 10 to 0.30 × 10⁵ ppm, preferably 0.10 × 10² to 0.30 × 10⁴ ppm.
(65) cyclo-arginyl-serine or a salt thereof, content: 0.40 × 10 to 0.70 × 10⁵ ppm, preferably 0.40 × 10² to 0.70 × 10⁴ ppm.
(66) cyclo-asparaginyl-methionine or a salt thereof, content: 0.40 × 10 to 0.70 × 10⁵ ppm, preferably 0.40 × 10² to 0.70 × 10⁴.
(67) cyclo-glutaminyl-methionine or a salt thereof, content: 0.10 × 10 to 0.20 × 10⁵ ppm, preferably 0.10 × 10² to 0.20 × 10⁴.
(68) cyclo-tryptophanyl-arginine or a salt thereof, content: 0.10 × 10 to 0.30 × 10⁵ ppm, preferably 0.10 × 10² to 0.30 × 10⁴.
(69) cyclo-asparaginyl-arginine or a salt thereof, content: 0.40 × 10 to 0.70 × 10⁵ ppm, preferably 0.40 × 10² to 0.70 × 10⁴ ppm.
(70) cyclo-asparaginyl-proline or a salt thereof, content: 0.05 × 10 to 0.09 × 10⁵ ppm, preferably 0.05 × 10² to 0.09 × 10⁴ ppm.
(71) cyclo-arginyl-arginine or a salt thereof, content: 0.40 × 10 to 0.70 × 10⁵ ppm, preferably 0.40 × 10² to 0.70 × 10⁴ ppm.

The composition according to the present invention is only required to contain one or two or more cyclic dipeptides of the above-mentioned 71 cyclic dipeptides in group A or salts thereof. Among them, one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, and cyclo-glutaminyl-isoleucine or salts thereof are preferably at least contained from the viewpoint of a GLP-1 secretion-accelerating action; hereinafter, the group of these 39 cyclic dipeptides is also referred to as "group B". More preferably, the composition according to the present invention contains one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, and cyclo-isoleucyl-lysine, each of which is a cyclic dipeptide including tyrosine as a constituent amino acid, or salts thereof; hereinafter, the group of these 10 cyclic dipeptides is also referred to as "group C".

The composition according to the present invention can accelerate GLP-1 secretion to ameliorate glucose metabolism due to a feature of containing the cyclic dipeptide or salt thereof in an effective amount, and thus can be suitably used for diseases for which amelioration of glucose metabolism is required. Examples of such diseases include diabetes and obesity, and the composition according to the present invention is effective for prevention and treatment of them.

The composition according to the present invention can be provided as a food with health claims or a health food for prevention or amelioration of the above diseases, for example, with a label indicating that this product is for prevention and/or amelioration of hyperglycemia and obesity, and is thus extremely useful for individuals with a high blood glucose level, slightly obese individuals, individuals with a tendency of metabolic syndrome, or the like.

Throughout the specification, a salt of a cyclic dipeptide refers to any pharmacologically acceptable salt (including inorganic salts and organic salts), and examples thereof include sodium salts, potassium salts, calcium salts, magnesium salts, ammonium salts, hydrochlorides, sulfates, nitrates, phosphates, and organic acid salts (acetate, citrates, maleates, malates, oxalates, lactates, succinate, fumarates, propionates, formates, benzoates, picrates, and benzenesulfonates) of the cyclic dipeptide.

Cyclic dipeptides used in the present invention can be prepared by using a method known in the art. For example, a cyclic dipeptide may be produced by using chemical synthesis, an enzymatic method, or microbial fermentation, or may be synthesized by using a dehydration and cyclization reaction of a linear peptide, or may be prepared in accordance with a method described in Japanese Patent Laid-Open No. 2003-252896 or J. Peptide Sci., 10, 737-737 (2004). For example, a heat-treated product obtained by subjecting a soybean peptide or tea peptide obtained through enzyme treatment or heat treatment to further heat treatment can be preferably used.
In other words, the cyclic dipeptide including amino acids as constituent units in the present invention may be derived from soybean or tea.

Specifically, a heat-treated product obtained by subjecting a solution containing a soybean peptide to heat treatment can be prepared, for example, by dissolving a soybean peptide in water to a concentration of 20 to 500 g/mL and heating the resultant under a condition of 40 to 150°C for 5 minutes to 120 hours. As desired, the heat-treated product obtained may be subjected to a process such as filtration, centrifugation, concentration, ultrafiltration, freeze-drying, and pulverization.

Specifically, a heat-treated product obtained by subjecting a solution containing a tea peptide to heat treatment can be prepared, for example, by dissolving a tea peptide in water to a concentration of 20 to 500 g/mL and heating the resultant under a condition of 40 to 150°C for 5 minutes to 120 hours. As desired, the heat-treated product obtained may be subjected to a process such as filtration, centrifugation, concentration, ultrafiltration, freeze-drying, and pulverization.

Those skilled in the art could easily prepare a salt of a cyclic dipeptide by using a method known in the art.

The composition according to the present invention contains the cyclic dipeptide or salt thereof in a specific amount, and exemplary embodiments thereof include three embodiments according to application of the composition described below. The content of the cyclic dipeptide or salt thereof in each embodiment can be measured in accordance with a known method, for example, by using LC-MS/MS.

### <Embodiment 1>

Embodiment 1 is an extract composition containing the cyclic dipeptide or salt thereof. The extract composition refers to, for example, a composition containing a preparation, as it is, obtained by treating a material containing the cyclic dipeptide or constituent amino acids of the cyclic dipeptide, or a composition containing a product obtained by diluting, concentrating, or purifying the preparation in accordance with a known method, and the extract composition may be formulated as it is or may be used for a raw material of a drug, a quasi-drug, a food or drink (health food), or the like.

For the content of each cyclic dipeptide or salt thereof in Embodiment 1, see the above description.

The number of components satisfying the above-mentioned content is one or more, preferably three or more, more preferably seven or more, and further preferably 11 or more.

The content of each component of the cyclic dipeptide or salt thereof in Embodiment 1 is as described above, and the total content of the cyclic dipeptide or salt thereof is preferably 0.2 × 10 ppm or more, more preferably 0.2 × 10² ppm or more, further preferably 0.4 × 10² ppm or more and preferably 1.0 × 10⁶ ppm or less, more preferably 1.0 × 10⁵ and preferably 1.0 × 10⁶ ppm or less, more preferably 1.0 × 10⁵ ppm or less, further preferably 0.5 × 10⁵ ppm or less.

The total content of cyclic dipeptides of group B, each of which has a high GLP-1 secretion-accelerating action, more preferably the total content of cyclic dipeptides of group C is preferably 0.2 × 10 ppm or more, more preferably 0.2 × 10² ppm or more, further preferably 0.4 × 10² ppm or more and preferably 1.0 × 10⁶ ppm or less, more preferably 1.0 × 10⁵ ppm or less, further preferably 0.5 × 10⁵ ppm or less.

The fraction of the total amount of cyclic dipeptides of group B or group C, each of which has a high GLP-1 secretion-accelerating action, in the cyclic dipeptide in Embodiment 1 is preferably 5% by weight or more, more preferably 10% by weight or more, and further preferably 15% by weight or more, from the viewpoint of a GLP-1 secretion-accelerating effect. The upper limit is not particularly limited, but preferably 90% by weight or less and more preferably 60% by weight or less from the viewpoint of solubilizing performance.

For example, a solvent, a dispersant, an emulsifier, a buffer, a stabilizer, a diluent, a binder, a disintegrator, a lubricant, etc., can be added to a raw material containing the cyclic dipeptide or salt thereof of the composition of Embodiment 1, as desired, to formulate a solid preparation such as a tablet, a granule, a powder (sanzai), a powder (funmatsuzai), and a capsule, or a solution such as a common solution, a suspension, and an emulsion by using a known method. Each of these compositions can be ingested as it is together with water or the like. In addition, the composition of Embodiment 1 can be prepared in a form capable of being mixed easily (e.g., form of a powder or a granule) and used for a raw material of a drug, a quasi-drug, a food or drink (health food), or the like.

Specific examples of the composition of Embodiment 1 include solid preparations such as tablets, granules, powders, and capsules, and solutions such as solutions, suspensions, and emulsions.

### <Embodiment 2>

Embodiment 2 is a drink composition containing the cyclic dipeptide or salt thereof. The drink composition refers to a composition primarily for drinking.

The number of components satisfying the above-mentioned content is one or more, preferably three or more, more preferably seven or more, and further preferably 11 or more.

The content of each component of the cyclic dipeptide or salt thereof in Embodiment 2 is as described above, and the total content of the cyclic dipeptide or salt thereof is preferably 0.2 × 10⁻² mg/100 mL or more, more preferably 0.2 × 10⁻² mg/100 mL or more, further preferably 0.4 × 10⁻² mg/100 mL or more and preferably 1.0 × 10³ mg/100 mL or less, more preferably 1.0 × 10² mg/100 mL or less, further preferably 0.5 × 10² mg/100 mL or less.

The total content of cyclic dipeptides of group B, each of which has a high GLP-1 secretion-accelerating action, more preferably the total content of cyclic dipeptides of group C is preferably 0.2 × 10⁻² mg/100 mL or more, more preferably 0.2 × 10⁻² mg/100 mL or more, further preferably 0.4 × 10⁻² mg/100 mL or more and preferably 1.0 × 10³ mg/100 mL or less, more preferably 1.0 × 10² mg/100 mL or less, further preferably 0.5 × 10² mg/100 mL or less.

The fraction of the total amount of cyclic dipeptides of group B or group C, each of which has a high GLP-1 secretion-accelerating action, in the cyclic dipeptide in Embodiment 2 is preferably 5% by weight or more, more preferably 10% by weight or more, and further preferably 15% by weight or more, from the viewpoint of a GLP-1 secretion-accelerating effect. The upper limit is not particularly limited, but preferably 90% by weight or less and more preferably 60% by weight or less from the viewpoint of solubilizing performance.

The composition of Embodiment 2, for example, a known drink composition may be prepared by mixing the cyclic dipeptide or salt thereof in a predetermined amount with raw materials of a known drink composition in accordance with a known production method for a drink composition, or may be prepared by adding the cyclic dipeptide or salt thereof to a known ready-made drink composition so as to reach the predetermined amount followed by dissolving and/or suspending. A known drink composition originally containing the cyclic dipeptide or salt thereof may be used, and it may be appropriately mixed for preparation as long as the amount of the cyclic dipeptide according to the present invention reaches the predetermined amount.

Specific examples of the composition of Embodiment 2 include non-alcohol drinks such as oolong tea drinks, tea drinks, green tea drinks, fruit juice drinks, vegetable juices, sports drinks, isotonic drinks, enhanced waters, mineral waters, near waters, coffee drinks, energy drinks, beauty drinks, and non-alcohol beer taste drinks, and alcohol drinks such as beer, wine, sake, plum liquor, low-malt beer, whisky, brandy, distilled spirits, rum, gin, and liquors. In the composition of Embodiment 2, one additive may be mixed singly or a plurality of additives may be mixed in combination, and examples of the additive include antioxidants, fragrances, organic acids, organic acid salts, inorganic acids, inorganic acid salts, inorganic salts, pigments, emulsifiers, preservatives, seasonings, sweeteners, acidulants, gums, oils, vitamins, amino acids, fruit juice extracts, vegetable extracts, pH adjusting agents, and quality stabilizers.

### <Embodiment 3>

Embodiment 3 is a food composition containing the cyclic dipeptide or salt thereof. The food composition refers to a composition primarily for ingestion of a meal or a snack.

The number of components satisfying the above-mentioned content is one or more, more preferably three or more, further preferably 39 or more, and particularly preferably 71 or more. Specifically, the content of cyclic dipeptides of group A is preferably within the above range, more preferably the content of cyclic dipeptides of group B is within the above range, and further preferably the content of cyclic dipeptides of group C is within the above range.

The content of each component of the cyclic dipeptide or salt thereof in Embodiment 3 is as described above, and the total content of the cyclic dipeptide or salt thereof is preferably 100% by weight or less, more preferably 80% by weight or less, and further preferably 60% by weight or less.

The total content of cyclic dipeptides of group B or group C, each of which has a high GLP-1 secretion-accelerating action, and salts thereof in Embodiment 3 is preferably 0.20 × 10⁻³% by weight or more, more preferably 0.20 × 10⁻²% by weight or more, further preferably 0.40 × 10⁻²% by weight or more and preferably 100% by weight or less, more preferably 80% by weight or less, further preferably 60% by weight or less.

The fraction of the total amount of cyclic dipeptides of group B or group C, each of which has a high GLP-1 secretion-accelerating action, and salts thereof in the cyclic dipeptide in Embodiment 3 is preferably 5% by weight or more, more preferably 10% by weight or more, and further preferably 15% by weight or more from the viewpoint of a GLP-1 secretion-accelerating action. The upper limit is not particularly limited, but preferably 90% by weight or less and more preferably 60% by weight or less from the viewpoint of solubilizing performance.

The composition of Embodiment 3, for example, a known food composition may be prepared by mixing the cyclic dipeptide or salt thereof in a predetermined amount with raw materials of a known food composition in accordance with a known production method for a food composition, or may be prepared by adding the cyclic dipeptide or salt thereof to a known ready-made food composition so as to reach the predetermined amount. A known food composition originally containing the cyclic dipeptide or salt thereof may be used, and it may be appropriately mixed for preparation as long as the amount of the cyclic dipeptide according to the present invention reaches the predetermined amount.

Specific examples of the composition of Embodiment 3 include boil-in-the-bag foods, seasonings (e.g., sources, soups, dressings, mayonnaises, creams), sweets (e.g., baked sweets such as pastries, cakes, cookies, and biscuits; chewing gums; chocolates; candies), and desserts (e.g., jellies, yogurts, ice creams). The form of a food or drink is not particularly limited, and may be any easy-to-ingest form such as a solid, a powder, a liquid, a gel, and a slurry. The composition of Embodiment 3 may be mixed in a dish containing an ingredient with a high purine content such as livers and sea foods including soft roe, shrimps, sardines, and skipjack tunas, or a dish suitable for beer taste drinks such as Chinese dumplings, Japanese meet and potato stews, braised pork stews, and hamburgers.

The composition according to the present invention can be ingested by using an appropriate method in accordance with its form. The method of ingestion is not particularly limited and may be any method which allows the cyclic dipeptide or salt thereof according to the present invention to be transferred into the circulating blood. Throughout the specification, ingestion includes all modes of eating, administration, and drinking.

The amount of ingestion of the composition according to the present invention is not constant, and is appropriately set in accordance with its form, administration method, intended use, and the age, body weight, and symptoms of a patient or patient animal as a subject to ingest. In the present invention, for example, the effective amount of ingestion of the cyclic dipeptide or salt thereof according to the present invention for a human is, in the case of a human with a body weight of 50 kg, preferably 0.2 mg or more, more preferably 2 mg or more, further preferably 20 mg or more and preferably 10 g or less, more preferably 5 g or less, further preferably 2 g or less per day. Administration may be performed in a single administration or multiple administrations in a day, within a desired dose range. Any duration may be used for the administration. Here, the effective amount of ingestion of the cyclic dipeptide or salt thereof according to the present invention for a human refers to the total amount of ingestion of the cyclic dipeptide or salt thereof which provides an effective action on a human, and the type of the cyclic dipeptide is not particularly limited.

Throughout the specification, the subject to ingest the composition according to the present invention is preferably a human, but may be a domestic animal such as a cattle, a horse, and a goat, a pet animal such as a dog, a cat, and a rabbit, or a laboratory animal such as a mouse, a rat, a guinea pig, and a monkey.

### EXAMPLES

The present invention will now be described with reference to Examples, but is not limited to the following Examples.

### <Reagents>

Cyclic dipeptides were synthesized by KNC Laboratories Co., Ltd. Used were a Poly-L-Lysine 96-well plate manufactured by BD Biosciences; PBS(+), an antibiotic, a Dulbecco's Modified Eagle's Medium (DMEM), glucose, and sodium carboxymethylcellulose (CMC-Na) manufactured by NACALAI TESQUE, Inc.; TPA manufactured by Cell Signaling Technology, Inc.; an active GLP-1 ELISA kit manufactured by Merck Millipore Corporation; Fetal bovine serum (FBS) manufactured by Sigma-Aldrich Co., LLC.; Sitagliptin phosphate manufactured by Santa Cruz Biotechnology, Inc.; a Glutest Neo Super and a Glutest Neo Sensor manufactured by SANWA KAGAKU KENKYUSHO CO., LTD.; soybean peptides (HINUTE AM) manufactured by Fuji Oil Co., Ltd.; and NCI-H716 cells donated by ATCC.

### <Statistical analysis>

In the Test Examples below, data were represented as average value ± standard error. In Test Examples 1, 2, and 3, a Student's t-test was used for a statistical test, and in other Test Examples, one-way ANOVA was carried out for dispersion analysis followed by a multiple comparison test by using a Dunnet's test. "*" and "#" in the results indicate the presence of a significant difference at p < 0.05 and the presence of a significant difference at p < 0.1, respectively. All of these analyses were carried out by using an SPSS for Windows release 17.0 (manufactured by SPSS Inc.).

### Test Example 1 (Study of in vitro GLP-1 secretion-accelerating action by using cyclic dipeptide)

Among the cyclic dipeptides, 92 highly water-soluble cyclic dipeptides were used in the following experiment. In each well of a Poly-L-Lysine 96-well plate, NCI-H716 cells suspended in 100 µL of a DMEM (with 10% FBS, 2 mM glutamine, and 1% antibiotics added in advance) were seeded at 0.5 × 10⁵ cells/well, and cultured in a CO₂ incubator (manufactured by ESPEC CORP.) for 48 hours. After washing with PBS(+), 100 µL of a PBS(+) solution with each cyclic dipeptide at a final concentration of 10 mM and 10 µM of Sitagliptin phosphate added therein was added to the cells. After 1 hour, the resultant solution was recovered, and the amount of active GLP-1 in the solution was measured by using the ELISA kit. In the analysis, the amount of active GLP-1 for a group with no cyclic dipeptide added was defined as 100, and relative values thereto were used.

Tables 1 to 5 show the results. In each Table, cyclic dipeptides for which an in vitro active GLP-1 secretion-accelerating activity was found and was not found are listed.

**[Table 1]**

| Experiment 1 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 23 | | |
| 1 | Cyclo(Ser-Ser) | 124 | 17 | 0.221 | No |
| 3 | Cyclo(Ala-Ala) | 95 | 7 | 0.418 | No |
| 5 | Cpclo(D-Ala-Pro) | 113 | 19 | 0.347 | No |
| 6 | Cyclo(Pro-Pro) | 87 | 20 | 0.348 | No |
| 7 | Cyclo(Phe-Ser) | 106 | 24 | 0.435 | No |
| 10 | Cyclo(Val-Pro) | 95 | 21 | 0.440 | No |
| 18 | Cyclo(Met-Pro) | 338 | 45 | 0.005 | Yes |
| 19 | Cyclo(Leu-Pro) | 173 | 16 | 0.031 | Yes |
| 25 | Cyclo(Ala-Ser) | 257 | 56 | 0.031 | Yes |
| 31 | Cyclo(Pro-Thr) | 204 | 32 | 0.029 | Yes |
| 34 | Cyclo(Ala-Hís) | 211 | 56 | 0.070 | No |
| 37 | Cyclo(His-Pro) | 231 | 88 | 0.113 | No |
| 40 | Cyclo(Lys-Lys) | 164 | 73 | 0.225 | No |
| 43 | Cyclo(Arg-Leu) | 226 | 24 | 0.010 | Yes |
| 45 | Cyclo(Glu-Pro) | 145 | 5 | 0.066 | No |

**[Table 2]**

| Experiment 2 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 6 | | |
| 46 | Cyclo(Ser-Pro) | 139 | 36 | 0.173 | No |
| 47 | Cyclo(Ile-Pro) | 79 | 16 | 0.146 | No |
| 48 | Cyclo(Asp-Pro) | 199 | 15 | 0.002 | Yes |
| 57 | Cyclo(Trp-Pro) | 81 | 11 | 0.111 | No |
| 58 | Cyclo(Lys-Pro) | 91 | 14 | 0.288 | No |
| 59 | Cyclo(Trp-Lys) | 239 | 48 | 0.022 | Yes |
| 60 | Cyclo(Trp-His) | 260 | 32 | 0.004 | Yes |
| 63 | Cyclo(Lys-Phe) | 220 | 20 | 0.002 | Yes |
| 65 | Cyclo(Gln-Phe) | 227 | 41 | 0.019 | Yes |
| 68 | Cyclo(Leu-Lys) | 208 | 51 | 0.053 | No |
| 69 | Cyclo(Ala-Lys) | 161 | 56 | 0.168 | No |
| 73 | Cyclo(Hyp-Gly) | 121 | 18 | 0.171 | No |
| 74 | Cyclo(Hyp-Pro) | 115 | 30 | 0.325 | No |
| 78 | Cyclo(Trp-Ser) | 119 | 32 | 0.301 | No |

**[Table 3]**

| Experiment 3 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 7 | | |
| 96 | Cyclo(Lys-Gly) | 133 | 13 | 0.046 | Yes |
| 97 | Cyclo(Glu-Lys) | 66 | 25 | 0.131 | No |
| 98 | Cyclo(Ser-Lvs) | 71 | 7 | 0.023 | Yes |
| 99 | Cyclo(Val-Lys) | 532 | 21 | 0.000 | Yes |
| 100 | Cyclo(Asp-Lys) | 116 | 42 | 0.360 | No |
| 101 | Cyclo(Asn-Lys) | 250 | 64 | 0.040 | Yes |
| 102 | Cyclo(Gln-Lys) | 112 | 12 | 0.228 | No |
| 107 | Cyclo(His-His) | 182 | 14 | 0.003 | Yes |
| 109 | Cyclo(Thr-His) | 290 | 7 | 0.000 | Yes |
| 110 | Cyclo(Asp-His) | 340 | 7 | 0.000 | Yes |
| 111 | Cyclo(Asn-His) | 341 | 33 | 0.001 | Yes |
| 112 | Cyclo(Arg-His) | 106 | 47 | 0.450 | No |
| 113 | Cyclo(Glu-Ala) | 157 | 36 | 0.100 | No |
| 114 | Cyclo(Thr-Ala) | 89 | 18 | 0.300 | No |
| 116 | Cyclo(Thr-Gly) | 146 | 36 | 0.140 | No |
| 117 | Cyclo(Arg-Gly) | 98 | 16 | 0.466 | No |
| 121 | Cyclo(Gly-Ala) | 153 | 61 | 0.221 | No |
| 124 | Cyclo(Asn-Ala) | 116 | 9 | 0.122 | No |
| 126 | Cyclo(Gln-Gly) | 210 | 48 | 0.044 | Yes |
| 129 | Cyclo(Asn-Glu) | 118 | 9 | 0.109 | No |
| 130 | Cyclo(Gln-Glu) | 120 | 8 | 0.078 | No |
| 136 | Cyclo(Cln-Ser) | 251 | 79 | 0.066 | No |
| 143 | Cyclo(Thr-Lys) | 105 | 21 | 0.413 | No |
| 153 | Cyclo(Asp-Ala) | 94 | 14 | 0.362 | No |
| 154 | Cyclo(Ser-Gly) | 116 | 2 | 0.053 | No |
| 157 | Cyclo(Thr-Glu) | 139 | 25 | 0.108 | No |
| 159 | Cyclo(Arg-Ser) | 135 | 14 | 0.048 | Yes |
| 164 | Cyclo(Thr-Thr) | 162 | 23 | 0.032 | Yes |
| 165 | Cyclo(Asp-Thr) | 140 | 28 | 0.121 | No |
| 166 | Cyclo(Asn-Thr) | 149 | 52 | 0.200 | No |
| 167 | Cyclo(Gln-Thr) | 156 | 61 | 0.204 | No |
| 168 | Cyclo(Arg-Thr) | 142 | 58 | 0.255 | No |
| 170 | Cyclo(Asn-Asp) | 125 | 13 | 0.089 | No |
| 171 | Cyclo(Gln-Asp) | 120 | 21 | 0.214 | No |

**[Table 4]**

| Experiment 4 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 27 | | |
| 172 | Cyclo(Met-Asp) | 141 | 40 | 0.220 | No |
| 173 | Cyclo(Asn-Asn) | 255 | 19 | 0.005 | Yes |
| 174 | Cyclo(Gln-Asn) | 305 | 64 | 0.021 | Yes |
| 175 | Cyclo(Asn-Met) | 218 | 53 | 0.059 | Yes |
| 176 | Cyclo(Gln-Gln) | 111 | 22 | 0.379 | No |
| 182 | Cyclo(Gln-Ile) | 445 | 51 | 0.002 | Yes |
| 186 | Cyclo(Gln-Met) | 191 | 7 | 0.015 | Yes |
| 187 | Cyclo(L-Ala-Pro) | 1186 | 171 | 0.002 | Yes |
| 188 | Cyclo(Gln-Pro) | 132 | 5 | 0.153 | No |
| 189 | Cyclo(Trp-Arg) | 314 | 39 | 0.005 | Yes |
| 191 | Cyclo(Gln-His) | 743 | 102 | 0.002 | Yes |
| 192 | Cyclo(Ser-Glu) | 993 | 77 | 0.000 | Yes |
| 193 | Cyclo(Asp-Ser) | 509 | 40 | 0.001 | Yes |
| 194 | Cyclo(Arg-Val) | 498 | 45 | 0.001 | Yes |
| 197 | Cyclo(Asn-Arg) | 359 | 73 | 0.015 | Yes |
| 199 | Cyclo(Met-Arg) | 652 | 73 | 0.001 | Yes |
| 201 | Cyclo(Asn-Pro) | 430 | 73 | 0.007 | Yes |
| 207 | Cyclo(Arg-Asp) | 1255 | 61 | 0.000 | Yes |
| 209 | Cyclo(Arg-Pro) | 441 | 29 | 0.000 | Yes |
| 212 | Cyclo(Arg-Arg) | 1050 | 58 | 0.000 | Yes |
| 215 | Cyclo(Asn-Gly) | 611 | 49 | 0.000 | Yes |

**[Table 5]**

| Experiment 5 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 6 | 0.086 | No |
| 2 | Cyclo(Gly-Gly) | 151 | 32 | 0.095 | No |
| 4 | Cyclo(Gly-Pro) | 157 | 125 | 0.044 | Yes |
| 21 | Cyclo(Gly-Leu) | 131 | 24 | 0.141 | No |
| 23 | Cyclo(Ald-Gln) | 99 | 26 | 0.480 | No |
| 24 | Cyclo(Glu-Gly) | 89 | 15 | 0.267 | No |
| 27 | Cyclo(Gly-His) | 82 | 20 | 0.216 | No |
| 32 | Cyclo(Asp-Gly) | 12 | 3 | 0.000 | Yes |
| 36 | Cyclo(Gln-Gly) | 75 | 15 | 0.099 | No |

### Test Example 2 (Study of in vivo plasma GLP-1 level-increasing action by using cyclic dipeptide)

Among the cyclic dipeptides, cyclic dipeptides for which an in vitro GLP-1 secretion-accelerating action was found and highly lipid-soluble cyclic dipeptides were used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a cyclic dipeptide dissolved or suspended in a 0.5% CMC-Na aqueous solution in 10 mg/kg was orally administered to each mouse forcibly. After 30 minutes, the blood was collected from the abdominal vena cava under anesthesia, and 1 µL of heparin sodium and 1 µL of 10 mM Sitagliptin phosphate were added to each collected blood, which was then subjected to centrifugation at 8000 rpm for 10 minutes to recover the plasma, and the plasma active GLP-1 level was measured by using the ELISA kit. In the analysis, the active GLP-1 level for a group administered with 0.5% CMC-Na aqueous solution was defined as 100, and relative values thereto were used.

Tables 6 to 15 show the results. In each Table, cyclic dipeptides for which an in vivo plasma active GLP-1 level-increasing action was found and was not found are listed.

**[Table 6]**

| Experiment 1 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 18 | Cyclo(Met-Pro) | 138 | 10 | 0.024 | Yes |
| 25 | Cyclo(Ala-Ser) | 158 | 10 | 0.007 | Yes |
| 31 | Cyclo(Pro-Thr) | 129 | 10 | 0.051 | No |
| 34 | Cyclo(Ala-His) | 100 | 67 | 0.500 | No |
| 37 | Cyclo(His-Pro) | 62 | 0 | 0.008 | Yes |
| 43 | Cyclo(Arg-Leu) | 138 | 10 | 0.024 | Yes |
| 48 | Cyclo(Asp-Pro) | 110 | 10 | 0.259 | No |
| 54 | Cyclo(Trp-Lys) | 119 | 0 | 0.058 | No |
| 60 | Cyclo(Trp-His) | 129 | 10 | 0.051 | No |
| 63 | Cyclo(Lvs-Phe) | 206 | 44 | 0.039 | Yes |

**[Table 7]**

| Experiment 2 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 182 | Cyclo(Gln-Ile) | 188 | 10 | 0.002 | Yes |
| 187 | Cyclo(L-Ala-Pro) | 217 | 10 | 0.001 | Yes |
| 191 | Cyclo(Gln-His) | 247 | 10 | 0.000 | Yes |
| 192 | Cyclo(Ser-Glu) | 286 | 17 | 0.000 | Yes |
| 193 | Cyclo(Asp-Ser) | 266 | 10 | 0.000 | Yes |
| 194 | Cyclo(Arg-Val) | 237 | 20 | 0.002 | Yes |
| 199 | Cyclo(Met-Arg) | 217 | 10 | 0.001 | Yes |
| 207 | Cyclo(Arg-Asp) | 266 | 26 | 0.002 | Yes |
| 209 | Cyclo(Arg-Pro) | 315 | 29 | 0.001 | Yes |
| 215 | Cyclo(Asn-Gly) | 305 | 10 | 0.000 | Yes |

**[Table 8]**

| Experiment 3 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 17 | | |
| 17 | Cyclo(Phe-Phe) | 203 | 9 | 0.003 | Yes |
| 22 | Cyclo(Trp-Tyr) | 203 | 9 | 0.003 | Yes |
| 26 | Cyclo(Gly-Trp) | 264 | 17 | 0.001 | Yes |
| 28 | Cyclo(Phe-Trp) | 255 | 17 | 0.002 | Yes |
| 38 | Cyclo(His-Phe) | 264 | 9 | 0.001 | Yes |
| 39 | Cyclo(Leu-Trp) | 229 | 9 | 0.001 | Yes |
| 44 | Cyclo(Ala-Leu) | 255 | 9 | 0.001 | Yes |
| 61 | Cyclo(Trp-Val) | 117 | 0 | 0.187 | No |
| 62 | Cyclo(Trp-Ile) | 178 | 71 | 0.173 | No |
| 75 | Cyclo(Trp-Trp) | 229 | 34 | 0.014 | Yes |

**[Table 9]**

| Experiment 4 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 8 | Cyclo(Gly-Phe) | 85 | 5 | 0.125 | No |
| 11 | Cyclo(Val-Val) | 100 | 13 | 0.500 | No |
| 12 | Cyclo(Met-Met) | 110 | 10 | 0.259 | No |
| 13 | Cyclo(Phe-Pro) | 120 | 9 | 0.103 | No |
| 14 | Cyclo(Ile-Ile) | 105 | 17 | 0.407 | No |
| 15 | Cyclo(Leu-Leu) | 95 | 10 | 0.371 | No |
| 16 | Cyclo(Leu-Phe) | 105 | 15 | 0.398 | No |
| 29 | Cyclo(Ser-Tyr) | 95 | 5 | 0.339 | No |
| 30 | Cyclo(Pro-Tyr) | 95 | 10 | 0.371 | No |
| 33 | Cyclo(Asp-Phe) | 90 | 9 | 0.246 | No |

**[Table 10]**

| Experiment 5 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 6 | | |
| 42 | Cyclo(Tyr-Gly) | 97 | 3 | 0.322 | No |
| 49 | Cyclo(Tyr-Tyr) | 100 | 0 | 0.500 | No |
| 52 | Cyclo(Ala-Phe) | 93 | 3 | 0.187 | No |
| 53 | Cyclo(Glu-Phe) | 93 | 3 | 0,187 | No |
| 54 | Cyclo(Val-Phe) | 93 | 9 | 0.281 | No |
| 55 | Cyclo(Ile-Phe) | 90 | 6 | 0.144 | No |
| 56 | Cyclo(Thr-Phe) | 87 | 3 | 0.058 | No |
| 64 | Cyclo(Asn-Phe) | 93 | 3 | 0.187 | No |
| 67 | Cyclo(Met-Phe) | 93 | 9 | 0.281 | No |
| 70 | Cyclo(Met-Lys) | 93 | 3 | 0.187 | No |

**[Table 11]**

| Experiment 6 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 0 | | |
| 71 | Cyclo(Ile-Leu) | 79 | 10 | 0.058 | No |
| 72 | Cyclo(Met-Leu) | 90 | 10 | 0.187 | No |
| 76 | Cyclo(Trp-Ala) | 110 | 10 | 0.187 | No |
| 77 | Cyclo(Trp-Glu) | 110 | 21 | 0.322 | No |
| 79 | Cyclo(Trp-Thr) | 100 | 18 | 0.500 | No |
| 81 | Cyclo(Trp-Asn) | 100 | 0 | 1.000 | No |
| 82 | Cyclo(Trp-Gln) | 90 | 10 | 0.187 | No |
| 83 | Cyclo(Trp-Met) | 110 | 10 | 0.187 | No |
| 85 | Cyclo(His-Tyr) | 90 | 10 | 0.187 | No |
| 86 | Cyclo(Ala-Tyr) | 90 | 21 | 0.322 | No |

**[Table 12]**

| Experiment 7 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 81 | | |
| 87 | Cyclo(Glu-Tyr) | 141 | 71 | 0.362 | No |
| 88 | Cyclo(Val-Tyr) | 385 | 71 | 0.029 | Yes |
| 89 | Cyclo(Ile-Tyr) | 222 | 108 | 0.208 | No |
| 90 | Cyclo(Thr-Tyr) | 466 | 108 | 0.027 | Yes |
| 91 | Cyclo(Asp-Tyr) | 181 | 81 | 0.259 | No |
| 92 | Cyclo(Asn-Tyr) | 181 | 41 | 0.211 | No |
| 93 | Cyclo(Gln-Tyr) | 344 | 41 | 0.028 | Yes |
| 94 | Cyclo(Arg-Tyr) | 222 | 41 | 0.125 | No |

**[Table 13]**

| Experiment 8 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | | 100 | 10 | | |
| 103 | Cyclo(His-Leu) | 94 | 12 | 0.362 | No |
| 105 | Cycio(Thr-Leu) | 94 | 6 | 0.322 | No |
| 106 | Cyclo(Asn-Leu) | 94 | 6 | 0.322 | No |
| 108 | Cyclo(Val-His) | 118 | 18 | 0.218 | No |
| 118 | Cyclo(Val-Glu) | 112 | 16 | 0.281 | No |
| 119 | Cyclo(Met-Ile) | 106 | 6 | 0.322 | No |
| 120 | Cyclo(Met-His) | 154 | 18 | 0.030 | Yes |
| 122 | Cyclo(Val-Ala) | 94 | 6 | 0.322 | No |

**[Table 14]**

| Experiment 9 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 7 | | |
| 123 | Cyclo(Ile-Ala) | 120 | 11 | 0.095 | No |
| 125 | Cyclo(Met-Ala) | 133 | 11 | 0.032 | Yes |
| 127 | Cyclo(Met-Gly) | 124 | 0 | 0.013 | Yes |
| 128 | Cyclo(Ile-Glu) | 133 | 4 | 0.008 | Yes |
| 131 | Cyclo(Met-Glu) | 137 | 0 | 0.003 | Yes |
| 132 | Cyclo(Val-Ser) | 129 | 4 | 0.012 | Yes |
| 133 | Cyclo(Ile-Ser) | 133 | 8 | 0.020 | Yes |
| 138 | Cyclo(Ile-Val) | 96 | 4 | 0.322 | No |
| 139 | Cyclo(Val-Thr) | 120 | 4 | 0.033 | Yes |
| 140 | Cyclo(Val-Asp) | 120 | 4 | 0.033 | Yes |

**[Table 15]**

| Experiment 10 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | | 100 | 23 | | |
| 141 | Cyclo(Asn-Val) | 191 | 23 | 0.024 | Yes |
| 145 | Cyclo(Glu-Leu) | 236 | 23 | 0.007 | Yes |
| 146 | Cyclo(Asp-Leu) | 259 | 23 | 0.004 | Yes |
| 148 | Cyclo(Ile-His) | 259 | 23 | 0.004 | Yes |
| 150 | Cyclo(Ile-Lys) | 282 | 0 | 0.001 | Yes |
| 151 | Cyclo(Ser-Leu) | 259 | 23 | 0.004 | Yes |
| 152 | Cyclo(Gln-Leu) | 282 | 0 | 0.001 | Yes |
| 155 | Cyclo(Val-Gly) | 55 | 91 | 0.326 | No |
| 156 | Cyclo(Ile-Gly) | 145 | 39 | 0.187 | No |
| 160 | Cyclo(Met-Val) | 304 | 60 | 0.017 | Yes |
| 161 | Cyclo(Ile-Thr) | 282 | 0 | 0.001 | Yes |
| 162 | Cyclo(Ile-Asp) | 259 | 45 | 0.018 | Yes |
| 163 | Cyclo(Asn-Ile) | 327 | 23 | 0.001 | Yes |
| 164 | Cyclo(Thr-Thr) | 282 | 0 | 0.001 | Yes |
| 169 | Cyclo(Met-Thr) | 282 | 0 | 0.001 | Yes |
| 173 | Cyclo(Asn-Asn) | 123 | 45 | 0.339 | No |
| 174 | Cyclo(Gln-Asn) | 168 | 23 | 0.051 | No |
| 177 | Cyclo(Ala-Cys) | 282 | 39 | 0.008 | Yes |
| 178 | Cyclo(Gly-Cys) | 282 | 0 | 0.001 | Yes |
| 179 | Cyclo(Glu-Cys) | 304 | 45 | 0.008 | Yes |
| 180 | Cyclo(Ser-Cys) | 259 | 23 | 0.004 | Yes |

### Test Example 3 (Study of in vivo plasma GLP-1 level-increasing action by using heat-treated soybean peptide)

A heat-treated soybean peptide was used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated soybean peptide dissolved in distilled water in 1 g/kg was orally administered to each mouse forcibly. After 15 minutes, 30 minutes, and 60 minutes, the blood was collected from the abdominal vena cava under anesthesia, and 1 µL of heparin sodium and 1 µL of 10 mM Sitagliptin phosphate were added to each collected blood, which was then subjected to centrifugation at 8000 rpm for 10 minutes to recover the plasma, and the plasma active GLP-1 level was measured by using the ELISA kit.

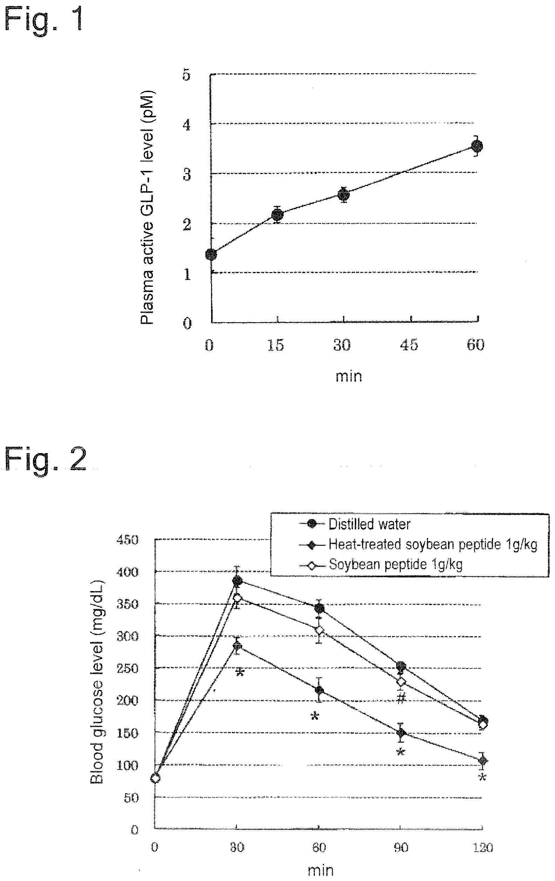

Fig. 1 shows the results.

### Test Example 4 (Study of in vivo glucose metabolism-enhancing action by using heat-treated soybean peptide)

A heat-treated soybean peptide and a soybean peptide were used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated soybean peptide or a soybean peptide dissolved in distilled water in 1 g/kg was orally administered to each mouse forcibly. To a control group, the equivalent amount of distilled water was orally administered forcibly. After 30 minutes, 1 g/kg of glucose was intraperitoneally administered to all of the groups. The blood was collected from the tail vein before the intraperitoneal administration of glucose and 30 minutes, 60 minutes, 90 minutes, and 120 minutes after the intraperitoneal administration of glucose, and the blood glucose level was measured by using a Glucose Neo Super and a Glucose Neo Sensor.

Fig. 2 shows the results.

### Test Example 5 (Study of in vivo glucose metabolism-enhancing action by using heat-treated tea peptide)

A heat-treated tea peptide was used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated tea peptide dissolved in distilled water in 1 g/kg was orally administered to each mouse forcibly. To a control group, the equivalent amount of distilled water was orally administered forcibly. After 30 minutes, 1 g/kg of glucose was intraperitoneally administered to all of the groups. The blood was collected from the tail vein before the intraperitoneal administration of glucose and 30 minutes, 60 minutes, 90 minutes, and 120 minutes after the intraperitoneal administration of glucose, and the blood glucose level was measured by using a Glucose Neo Super and a Glucose Neo Sensor.

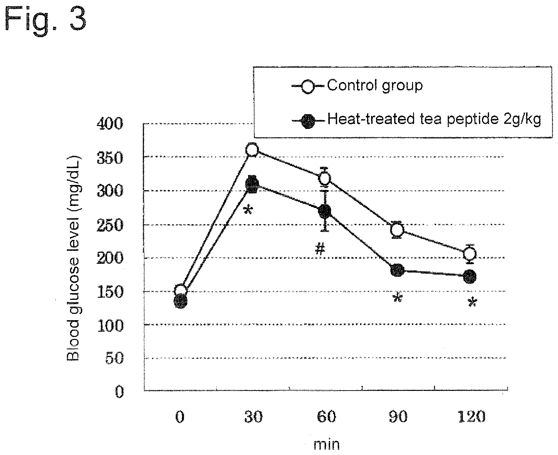

Fig. 3 shows the results.

### Production Examples

Hereinafter, specific formulations for compositions containing the cyclic dipeptide or salt thereof of the present invention will be illustrated. These compositions can be prepared in accordance with a known method.

### (Production Example) Production of carbonated drink

Synthetic cyclic dipeptides as raw materials at a formulation ratio listed in Table 16 are dissolved in water, and the pH was then adjusted to 3.8 with phosphoric acid. Thereto, an antioxidant, a fragrance, an acidulant, a sweetener, and a caramel coloring each in an appropriate amount are added and the resultant is stored for about 24 hours. During the storage, carbon dioxide gas in an appropriate amount is added thereto, and the resultant is then subjected to steps of filtration, bottling, and sterilizing (heating at 65°C or higher for 10 minutes) to afford a carbonated drink.

**[Table 16]**

| | Production Example (1) | Production Example (2) | Production Example (3) | Production Example (4) | Production Example (5) | Production Example (6) | Production Example (7) |
|---|---|---|---|---|---|---|---|
| Raw material name/product name | Amount added (% by weight) | | | | | | |
| Cyclo-threonyl-tyrosine | 1.0×10⁻² mg/100 ml | | | | | | |
| Cyclo-valyl-tyrosine | 2.0×10⁻² mg/100 ml | | | | | | |
| Cyclo-glutaminyl-tyrosine | 3.0×10⁻² mg/100 ml | | | | | | |
| Cyclo-asparaginyl-isoleucine | 2.0×10⁻² mg/100 ml | | | | | | |
| Cyclo-arginyl-proline | 2.0×10⁻² mg/100 ml | | | | | | |
| SOYAFIBE-S-LA200 (manufactured by Fuji Oil Co., Ltd.) | 0.8 | - | - | 0.4 | 0.4 | 0.4 | 0.2 |
| PINEUP (manufactured by Matsutani Chemical Industry Co., Ltd.) | 0 | 0.8 | - | 0.4 | 0.4 | - | 0.2 |
| SM900 (manufactured by San-Ei Gen F.F.I., Inc.) | 0 | - | 0.8 | - | - | 0.4 | 0.2 |
| HINUTE AM (manufactured by Fuji Oil Co., Ltd.) | 0.2 | 0.2 | 0.2 | 0.2 | 0.2 | 0.2 | 0.4 |

### (Production Example) Production example of chocolate

Raw materials at a formulation ratio listed in Table 17 are used for production of a chocolate. The raw materials listed in Table 17 are charged into a Hobart mixer, and mixed at an intermediate speed for 3 minutes, and the resultant is further subjected to rolling and conching to afford a chocolate dough. This chocolate dough is tempered and then cast in a mold, which is cooled to afford the chocolate according to the present invention.

**[Table 17]**

| | Production Example (8) | Production Example (9) | Production Example (10) | Production Example (11) | Production Example (12) |
|---|---|---|---|---|---|
| Raw material | Composition (% by weight) | | | | |
| Cyclo-threonyl-tyrosine | 0.05 | - | - | 0.05 | 0.05 |
| Cyclo-valyl-tyrosine | - | 0.05 | - | 0.05 | - |
| Cyclo-glutaminyl-tyrosine | - | - | 0.10 | - | 0.05 |
| Cyclo-asparaginyl-isoleucine | 0.05 | | | | |
| Cyclo-arginyl-proline | | 0.05 | | | |
| Cocoa mass | 18.7 | | | | |
| Cocoa butter | 26.5 | | | | |
| Sugar | 34.7 | | | | |

## Claims

1. A composition comprising a cyclic dipeptide including amino acids as constituent units or a salt thereof, wherein the total amount of the cyclic dipeptide including amino acids as constituent units or salt thereof is 0.2 × 10 to 1.0 × 10⁶ ppm.

2. The composition according to claim 1, wherein the cyclic dipeptide including amino acids as constituent units or salt thereof is one or two or more cyclic dipeptides or salts thereof of the following (1) to (71) each contained in an amount satisfying the content range:
(1) cyclo-threonyl-tyrosine or a salt thereof, content: 0.40 × 10 to 0.70 × 10⁵ ppm;
(2) cyclo-valyl-tyrosine or a salt thereof, content: 0.30 × 10 to 0.50 × 10⁵ ppm;
(3) cyclo-glutaminyl-tyrosine or a salt thereof, content: 0.20 × 10 to 0.40 × 10⁵ ppm;
(4) cyclo-asparaginyl-isoleucine or a salt thereof, content: 0.40 × 10 to 0.70 × 10⁵ ppm;
(5) cyclo-arginyl-proline or a salt thereof, content: 0.40 × 10 to 0.70 × 10⁵ ppm;
(6) cyclo-asparaginyl-glycine or a salt thereof, content: 0.20 × 10 to 0.50 × 10⁵ ppm;
(7) cyclo-methionyl-valine or a salt thereof, content: 0.40 × 10 to 0.70 × 10⁵ ppm;
(8) cyclo-glutamyl-cysteine or a salt thereof, content: 0.40 × 10 to 0.70 × 10⁵ ppm;
(9) cyclo-seryl-glutamic acid or a salt thereof, content: 0.40 × 10 to 0.70 × 10⁵ ppm;
(10) cyclo-isoleucyl-lysine or a salt thereof, content: 0.50 × 10 to 0.90 × 10⁵ pp;
(11) cyclo-glutaminyl-leucine or a salt thereof, content: 0.80 × 10 to 1.30 × 10⁵ ppm;
(12) cyclo-isoleucyl-threonine or a salt thereof, content: 0.20 × 10 to 0.50 × 10⁵ ppm;
(13) cyclo-threonyl-threonine or a salt thereof, content: 0.30 × 10 to 0.50 × 10⁵ ppm;
(14) cyclo-methionyl-threonine or a salt thereof, content: 0.40 × 10 to 0.70 × 10⁵ ppm;
(15) cyclo-alanyl-cysteine or a salt thereof, content: 0.40 × 10 to 0.70 × 10⁵ ppm;
(16) cyclo-glycyl-cysteine or a salt thereof, content: 0.40 × 10 to 0.70 × 10⁵ ppm;
(17) cyclo-aspartyl-serine or a salt thereof, content: 1.00 × 10 to 1.60 × 10⁵ ppm;
(18) cyclo-arginyl-aspartic acid or a salt thereof, content: 0.90 × 10 to 1.50 × 10⁵ ppm;
(19) cyclo-glycyl-tryptophan or a salt thereof, content: 0.10 × 10 to 0.30 × 10⁵ ppm;
(20) cyclo-histidyl-phenylalanine or a salt thereof, content: 0.20 × 10 to 0.40 × 10⁵ ppm;
(21) cyclo-aspartyl-leucine or a salt thereof, content: 0.40 × 10 to 0.80 × 10⁵ ppm;
(22) cyclo-isoleucyl-histidine or a salt thereof, content: 0.70 × 10 to 1.20 × 10⁵ ppm;
(23) cyclo-seryl-leucine or a salt thereof, content: 0.70 × 10 to 1.10 × 10⁵ ppm;
(24) cyclo-isoleucyl-aspartic acid or a salt thereof, content: 0.70 × 10 to 1.10 × 10⁵ ppm;
(25) cyclo-seryl-cysteine or a salt thereof, content: 0.40 × 10 to 0.70 × 10⁵ ppm;
(26) cyclo-phenylalanyl-tryptophan or a salt thereof, content: 0.07 × 10 to 0.20 × 10⁵ ppm;
(27) cyclo-alanyl-leucine or a salt thereof, content: 1.40 × 10 to 2.30 × 10⁵ ppm;
(28) cyclo-glutaminyl-histidine or a salt thereof, content: 0.40 × 10 to 0.70 × 10⁵ ppm;
(29) cyclo-arginyl-valine or a salt thereof, content: 0.60 × 10 to 1.00 × 10⁵ ppm;
(30) cyclo-glutamyl-leucine or a salt thereof, content: 0.70 × 10 to 1.10 × 10⁵ ppm;
(31) cyclo-leucyl-tryptophan or a salt thereof, content: 0.10 × 10 to 0.30 × 10⁵ ppm;
(32) cyclo-tryptophanyl-tryptophan or a salt thereof, content: 0.04 × 10 to 0.07 × 10⁵ ppm;
(33) cyclo-L-alanyl-proline or a salt thereof, content: 0.40 × 10 to 0.70 × 10⁵ ppm;
(34) cyclo-methionyl-arginine or a salt thereof, content: 0.20 × 10 to 0.50 × 10⁵ ppm;
(35) cyclo-lysyl-phenylalanine or a salt thereof, content: 1.00 × 10 to 1.60 × 10⁵ ppm;
(36) cyclo-phenylalanyl-phenylalanine or a salt thereof, content: 0.30 × 10 to 0.60 × 10⁵ ppm;
(37) cyclo-tryptophanyl-tyrosine or a salt thereof, content: 0.10 × 10 to 0.20 × 10⁵ ppm;
(38) cyclo-asparaginyl-valine or a salt thereof, content: 0.30 × 10 to 0.50 × 10⁵ ppm;
(39) cyclo-glutaminyl-isoleucine or a salt thereof, content: 0.50 × 10 to 0.80 × 10⁵ ppm;
(40) cyclo-alanyl-serine or a salt thereof, content: 0.40 × 10 to 0.80 × 10⁵ ppm;
(41) cyclo-methionyl-histidine or a salt thereof, content: 0.10 × 10 to 0.30 × 10⁵ ppm;
(42) cyclo-methionyl-proline or a salt thereof, content: 0.10 × 10 to 0.30 × 10⁵ ppm;
(43) cyclo-arginyl-leucine or a salt thereof, content: 1.50 × 10 to 2.30 × 10⁵ ppm;
(44) cyclo-methionyl-glutamic acid or a salt thereof, content: 0.10 × 10 to 0.30 × 10⁵ ppm;
(45) cyclo-methionyl-alanine or a salt thereof, content: 0.20 × 10 to 0.50 × 10⁵ ppm;
(46) cyclo-isoleucyl-glutamic acid or a salt thereof: 0.50 × 10 to 0.90 × 10⁵ ppm;
(47) cyclo-isoleucyl-serine or a salt thereof, content: 0.50 × 10 to 0.90 × 10⁵ ppm;
(48) cyclo-valyl-serine or a salt thereof, content: 0.50 × 10 to 0.90 × 10⁵ ppm;
(49) cyclo-methionyl-glycine or a salt thereof, content: 0.40 × 10 to 0.70 × 10⁵ ppm;
(50) cyclo-valyl-threonine or a salt thereof, content: 0.30 × 10 to 0.60 × 10⁵ ppm;
(51) cyclo-valyl-aspartic acid or a salt thereof, content: 0.50 × 10 to 0.90 × 10⁵ ppm;
(52) cycle-glycyl-proline or a salt thereof, content: 0.30 × 10 to 0.50 × 10⁵ ppm;
(53) cyclo-leucyl-proline or a salt thereof, content: 0.50 × 10 to 0.90 × 10⁵ ppm;
(54) cyclo-glutaminyl-glycine or a salt thereof, content: 0.05 × 10 to 0.09 × 10⁵ ppm;
(55) cyclo-tryptophanyl-lysine or a salt thereof, content: 0.20 × 10 to 0.40 × 10⁵ ppm;
(56) cyclo-glutaminyl-phenylalanine or a salt thereof, content: 0.30 × 10 to 0.60 × 10⁵ ppm;
(57) cyclo-lysyl-glycine or a salt thereof, content: 1.00 × 10 to 1.60 × 10⁵ ppm;
(58) cyclo-seryl-lysine or a salt thereof, content: 1.60 × 10 to 2.60 × 10⁵ ppm;
(59) cyclo-valyl-lysine or a salt thereof, content: 0.90 × 10 to 1.50 × 10⁵ ppm;
(60) cyclo-asparaginyl-lysine or a salt thereof, content: 0.60 × 10 to 1.10 × 10⁵ ppm;
(61) cyclo-histidyl-histidine or a salt thereof, content: 0.40 × 10 to 0.70 × 10⁵ ppm;
(62) cyclo-threonyl-histidine or a salt thereof, content: 0.40 × 10 to 0.70 × 10⁵ ppm;
(63) cyclo-aspartyl-histidine or a salt thereof, content: 0.40 × 10 to 0.70 × 10⁵ ppm;
(64) cyclo-asparaginyl-histidine or a salt Thereof, content: 0.10 × 10 to 0.30 × 10⁵ ppm;
(65) cyclo-arginyl-serine or a salt thereof, content: 0.40 × 10 to 0.70 × 10⁵ ppm;
(66) cyclo-asparaginyl-methionine or a salt thereof, content: 0.40 × 10 to 0.70 × 10⁵ ppm;
(67) cyclo-glutaminyl-methionine or a salt thereof, content: 0.10 × 10 to 0.20 × 10⁵ ppm;
(68) cyclo-tryptophanyl-arginine or a salt thereof, content: 0.10 × 10 to 0.30 × 10⁵ ppm;
(69) cyclo-asparaginyl-arginine or a salt thereof, content: 0.40 × 10 to 0.70 × 10⁵ ppm;
(70) cyclo-asparaginyl-proline or a salt thereof, content: 0.05 × 10 to 0.09 × 10⁵ ppm; and
(71) cyclo-arginyl-arginine or a salt thereof, content: 0.40 × 10 to 0.70 × 10⁵ ppm.

3. The composition according to claim 1 or 2, wherein one or two or more of the following (1) to (10) as the cyclic dipeptide including amino acids as constituent units or salt thereof are each comprised in the following content range:
(1) cyclo-threonyl-tyrosine or a salt thereof, content: 0.40 × 10 to 0.70 × 10⁵ ppm;
(2) cyclo-valyl-tyrosine or a salt thereof, content: 0.30 × 10 to 0.50 × 10⁵ ppm;
(3) cyclo-glutaminyl-tyrosine or a salt thereof, content : 0.20 × 10 to 0.40 × 10⁵ ppm;
(4) cyclo-asparaginyl-isoleucine or a salt thereof, content: 0.40 × 10 to 0.70 × 10⁵ ppm;
(5) cyclo-arginyl-proline or a salt thereof, content: 0.40 × 10 to 0.70 × 10⁵ ppm;
(6) cyclo-asparaginyl-glycine or a salt thereof, content: 0.20 × 10 to 0.50 × 10⁵ ppm;
(7) cyclo-methionyl-valine or a salt thereof, content: 0.40 × 10 to 0.70 × 10⁵ ppm;
(8) cyclo-glutamyl-cysteine or a salt thereof, content: 0.40 × 10 to 0.70 × 10⁵ ppm;
(9) cyclo-seryl-glutamic acid or a salt thereof, content: 0.40 × 10 to 0.70 × 10⁵ ppm; and
(10) cyclo-isoleucyl-lysine or a salt thereof, content: 0.50 × 10 to 0.90 × 10⁵ ppm.

4. A composition comprising a cyclic dipeptide including amino acids as constituent units or a salt thereof, wherein the total amount of the cyclic dipeptide including amino acids as constituent units or salt thereof is 0.2 × 10⁻² to 1.0 × 10³ mg/100 mL.

5. The composition according to claim 4, wherein the cyclic dipeptide including amino acids as constituent units or salt thereof is one or two or more cyclic dipeptides or salts thereof of the following (1) to (71) each contained in an amount satisfying the content range:
(1) cyclo-threonyl-tyrosine or a salt thereof, content: 0.40 × 10⁻² to 0.70 × 10² mag/100 mL;
(2) cyclo-valyl-tyrosine or a salt thereof, content: 0.30 × 10⁻² to 0.50 × 10² mg/100 mL;
(3) cyclo-glutaminyl-tyrosine or a salt thereof, content: 0.20 × 10⁻² to 0.40 × 10² mag/100 mL;
(4) cyclo-asparaginyl-isoleucine or a salt thereof, content: 0.40 × 10⁻² to 0.70 × 10² mg/100 mL;
(5) cyclo-arginyl-proline or a salt thereof, content: 0.40 × 10⁻² to 0.70 × 10² mg/100 mL;
(6) cyclo-asparaginyl-glycine or a salt thereof, content: 0.20 × 10⁻² to 0.50 × 10² mg/100 mL;
(7) cyclo-methionyl-valine or a salt thereof, content: 0.40 × 10⁻² to 0.70 × 10² mg/100 mL;
(8) cyclo-glutamyl-cysteine or a salt thereof, content: 0.40 × 10⁻² to 0.70 × 10² mg/100 mL;
(9) cyclo-seryl-glutamic acid or a salt thereof, content: 0.40 × 10⁻² to 0.70 × 10² mg/100 mL;
(10) cyclo-isoleucyl-lysine or a salt thereof, content: 0.50 × 10⁻² to 0.90 × 10² mg/100 mL;
(11) cyclo-glutaminyl-leucine or a salt thereof, content: 0.80 × 10⁻² to 1.30 × 10² mg/100 mL;
(12) cyclo-isoleucyl-threonine or a salt thereof, content: 0.20 × 10⁻² to 0.50 × 10² mg/100 mL;
(13) cyclo-threonyl-threonine or a salt thereof, content: 0.30 × 10⁻² to 0.5 × 10² mg/100 mL;
(14) cyclo-methionyl-threonine or a salt thereof, content: 0.4 × 10⁻² to 0.70 × 10² mg/100 mL;
(15) cyclo-alanyl-cysteine or a salt thereof, content: 0.40 × 10⁻² to 0.70 × 10² mg/100 mL;
(16) cyclo-glycy-cysteine or a salt thereof, content: 0.40 × 10⁻² to 0.70 0 × 10² mg/100 mL;
(17) cyclo-aspartyl-serine or a salt thereof, content: 1.00 × 10⁻² to 1.60 × 10² mg/100 mL;
(18) cyclo-arginyl-aspartic acid or a salt thereof, content: 0.90 × 10⁻² to 1.50 × 10² mg/100 mL;
(19) cyclo-glycyl-tryptophan or a salt thereof, content: 0.10 × 10⁻² to 0.30 × 10² mg/100 mL;
(20) cyclo-histidyl-phenylalanine or a salt thereof, content: 0.20 × 10⁻² to 0.40 × 10² mg/100 mL;
(21) cyclo-aspartyl-leucine or a salt thereof, content: 0.40 × 10⁻² to 0.80 × 10² mg/100 mL;
(22) cyclo-isoleucyl-histidine or a salt thereof, content: 0.70 × 10⁻² to 1.20 × 10² mg/100 mL;
(23) cyclo-seryl-leucine or a salt thereof, content: 0.70 × 10⁻² to 1.10 × 10² mag/100 mL;
(24) cyclo-isoleucyl-aspartic acid or a salt thereof, content: 0.70 × 10⁻² to 1.10 × 10² mg/100 mL;
(25) cyclo-seryl-cysteine or a salt thereof, content: 0.40 × 10⁻² to 0.70 × 10² mg/100 mL;
(26) cyclo-phenylalanyl-tryptophan or a salt thereof, content: 0.07 × 10⁻² to 0.20 × 10² mag/100 mL;
(27) cyclo-alanyl-leucine or a salt thereof, content: 1.40 × 10⁻² to 2.30 × 10² mg/100 mL;
(28) cyclo-glutaminyl-histidine or a salt thereof, content: 0.40 × 10⁻² to 0.70 × 10² mag/100 mL;
(29) cyclo-arginyl-valine or a salt thereof, content: 0.60 × 10 to 1.00 × 10² mg/100 mL;
(30) cyclo-glutamyl-leucine or a salt thereof, content: 0.70 × 10⁻² to 1.10 × 10² mg/100 mL;
(31) cyclo-leucyl-tryptophan or a salt thereof, content: 0.10 × 10⁻² to 0.30 × 10² mg/100 mL;
(32) cyclo-tryptophanyl-tryptophan or a salt thereof, content: 0.04 × 10⁻² to 0.07 × 10² mg/100 mL;
(33) cyclo-L-alanyl-proline or a salt thereof, content: 0.40 × 10⁻² to 0.70 × 10² mg/100 mL;
(34) cyclo-methionyl-arginine or a salt thereof, content: 0.20 × 10⁻² to 0.50 × 10² mg/100 mL;
(35) cyclo-lysyl-phenylalanine or a salt thereof, content: 1.00 × 10⁻² to 1.60 × 10² mg/100 mL;
(36) cyclo-phenylalanyl-phenylalanine or a salt thereof, content: 0.30 × 10⁻² to 0.60 × 10² mg/100 mL;
(37) cyclo-tryptophanyl-tyrosine or a salt thereof, content: 0.10 × 10⁻² to 0.20 × 10² mg/100 mL;
(38) cyclo-asparaginyl-valine or a salt thereof, content: 0.30 × 10⁻² to 0.50 × 10² mg/100 mL;
(39) cyclo-glutaminyl-isoleucine or a salt thereof, content: 0.50 × 10⁻² to 0.80 × 10² mg/100 mL;
(40) cyclo-alanyl-serine or a salt thereof, content: 0.40 × 10⁻² to 0.80 × 10² mg/100 mL;
(41) cyclo-methionyl-histidine or a salt thereof, content: 0.10 × 10⁻² to 0.30 × 10² mg/100 mL;
(42) cyclo-methionyl-proline or a salt thereof, content: 0.10 × 10⁻² to 0.30 × 10² mag/100 mL;
(43) cyclo-arginyl-leucine or a salt thereof, content: 1.50 × 10⁻² to 2.30 × 10² mg/100 mL;
(44) cyclo-methionyl-glutamic acid or a salt thereof, content: 0.10 × 10⁻² to 0.30 × 10² mg/100 mL;
(45) cyclo-methionyl-alanine or a salt thereof, content: 0.20 × 10⁻² to 0.50 × 10² mg/100 mL;
(46) cyclo-isoleucyl-glutamic acid or a salt thereof: 0.50 × 10⁻² to 0.90 × 10² mg/100 mL;
(47) cyclo-isoleucyl-serine or a salt thereof, content: 0.50 × 10² to 0.90 × 10² mg/100 mL;
(48) cyclo-valyl-serine or a salt thereof, content: 0.50 × 10⁻² to 0.90 × 10² mg/100 mL;
(49) cyclo-methlonyl-glycine or a salt thereof, content: 0.40 × 10⁻² to 0.70 × 10² mg/100 mL;
(50) cyclo-valyl-threonine or a salt thereof, content: 0.30 × 10⁻² to 0.60 × 10² mg/100 mL;
(51) cyclo-valyl-aspartic acid or a salt thereof, content: 0.50 × 10⁻² to 0.90 × 10² mg/100 mL;
(52) cyclo-glycyl-proline or a salt thereof, content: 0.30 × 10⁻² to 0.50 × 10² mg/100 mL;
(53) cyclo-leucyl-proline or a salt thereof, content: 0.50 × 10⁻² to 0.90 × 10² mg/100 mL;
(54) cyclo-glutaminyl-glycine or a salt thereof, content: 0.05 × 10⁻² to 0.09 × 10² mg/100 mL;
(55) cyclo-tryptophanyl-lysine or a salt thereof, content: 0.20 × 10⁻² to 0.40 × 10² mg/100 mL;
(56) cyclo-glutaminyl-phenylalanine or a salt thereof, content: 0.30 × 10⁻² to 0.60 × 10² mg/100 mL;
(57) cyclo-lysyl-glycine or a salt thereof, content: 1.00 × 10⁻² to 1.60 × 10² mag/100 mL;
(58) cyclo-seryl-lysine or a salt thereof, contend: 1.60 × 10⁻² to 2.60 × 10² mg/100 mL;
(59) cyclo-valyl-lysine or a salt thereof, content: 0.90 × 10⁻² to 1.50 × 10² mg/100 mL;
(60) cyclo-asparaginyl-lysine or a salt thereof, content: 0.60 × 10⁻² to 1.10 × 10² mg/100 mL;
(61) cyclo-histidyl-histidine or a salt thereof, content: 0.40 × 10⁻² to 0.70 × 10² mg/100 mL;
(62) cyclo-threonyl-histidine or a salt thereof, content: 0.40 × 10⁻² to 0.70 × 10² mg/100 mL;
(63) cyclo-aspartyl-histidine or a salt thereof, content: 0.40 × 10⁻² to 0.70 × 10² mg/100 mL;
(64) cyclo-asparaginyl-histidine or a salt thereof, content: 0.10 × 10⁻² to 0.30 × 10² mg/100 mL;
(65) cyclo-arginyl-serine or a salt thereof, content: 0.40 × 10⁻² to 0.70 × 10² mg/100 mL;
(66) cyclo-asparaginyl-methionine or a salt thereof, content: 0.40 × 10⁻² to 0.70 × 10² mag/100 mL;
(67) cyclo-glutaminyl-methionine or a salt thereof, content: 0.10 × 10⁻² to 0.20 × 10² mg/100 mL;
(68) cyclo-tryptophanyl-arginine or a salt thereof, content: 0.10 × 10⁻² to 0.30 × 10² mg/100 mL;
(69) cyclo-asparaginyl-arcinine or a salt thereof, content: 0.40 × 10⁻² to 0.70 × 10² mag/100 mL;
(70) cyclo-asparaginyl-proline or a salt thereof, content: 0.05 × 10⁻² to 0.09 × 10² mg/100 mL; and
(71) cyclo-arginyl-arginine or a salt thereof, content: 0.40 × 10⁻² to 0.70 × 10² mg/100 mL.

6. The composition according to claim 4 or 5, wherein one or two or more of the following (1) to (10) as the cyclic dipeptide including amino acids as constituent units or salt thereof are each comprised in the following content range:
(1) cyclo-threonyl-tyrosine or a salt thereof, content: 0.40 × 10⁻² to 0.70 × 10² mg/100 mL;
(2) cyclo-valyl-tyrosine or a salt thereof, content: 0.30 × 10⁻² to 0.50 × 10² mg/100 mL;
(3) cyclo-glutaminyl-tyrosine or a salt thereof, content: 0.20 × 10⁻² to 0.40 × 10² mag/100 mL;
(4) cyclo-asparaginyl-isoleucine or a salt thereof, content: 0.40 × 10⁻² to 0.70 × 10² mg/100 mL;
(5) cyclo-arginyl-proline or a salt thereof, content: 0.40 × 10⁻² to 0.70 × 10² mg/100 mL;
(6) cyclo-asparaginyl-glycine or a salt thereof, content: 0.20 × 10⁻² to 0.50 × 10² mg/100 mL;
(7) cyclo-methionyl-valine or a salt thereof, content: 0.40 × 10⁻² to 0.70 × 10² mag/100 mL;
(8) cyclo-glutamyl-cysteine or a salt thereof, content: 0.40 × 10⁻² to 0.70 × 10² mg/100 mL;
(9) cyclo-seryl-glutamic acid or a salt thereof, content: 0.40 × 10⁻² to 0.70 × 10² mg/100 mL; and
(10) cyclo-isoleucyl-lysine or a salt thereof, content: 0.50 × 10⁻² to 0.90 × 10² mg/100 mL.

7. A composition comprising a cyclic dipeptide including amino acids as constituent units or a salt thereof, wherein the total amount of the cyclic dipeptide including amino acids as constituent units or salt thereof is 0.20 × 10⁻³ to 100% by weight.

8. The composition according to claim 7, wherein the cyclic dipeptide including amino acids as constituent units or salt thereof is one or two or more cyclic dipeptides or salts thereof of the following (1) to (71) each contained in an amount satisfying the content range:
(1) cyclo-threonyl-tyrosine or a salt thereof, content: 0.40 × 10⁻³ to 0.70 × 10% by weight;
(2) cyclo-valyl-tyrosine or a salt thereof, content: 0.30 × 10⁻³ to 0.50 × 10% by weight;
(3) cyclo-glutaminyl-tyrosine or a salt thereof, content: 0.20 × 10⁻³ to 0.40 × 10% by weight;
(4) cyclo-asparaginyl-isoleucine or a salt thereof, content: 0.40 × 10⁻³ to 0.70 × 10° by weight;
(5) cyclo-arginyl-proline or a salt thereof, content: 0.40 × 10⁻³ to 0.70 × 10% by weight;
(6) cyclo-asparaginyl-glycine or a salt thereof, content: 0.20 × 10⁻³ to 0.50 × 10% by weight;
(7) cyclo-methionyl-valine or a salt thereof, content: 0.40 × 10⁻³ to 0.70 × 10% by weight;
(8) cyclo-glutamyl-cysteine or a salt thereof, content: 0.40 × 10⁻³ to 0.70 × 10% by weight;
(9) cyclo-seryl-glutamic acid or a salt thereof, content: 0.40 × 10⁻³ to 0.70 × 10% by weight;
(10) cyclo-isoleucyl-lysine or a salt thereof, content: 0.50 × 10⁻³ to 0.90 × 10% by weight;
(11) cyclo-glutaminyl-leucine or a salt thereof, content: 0.80 × 10⁻³ to 1.30 × 10% by weight;
(12) cyclo-isoleucyl-threonine or a salt thereof, content: 0.20 × 10⁻³ to 0.50 × 10% by weight;
(13) cyclo-threonyl-threonine or a salt thereof, content: 0.30 × 10⁻³ to 0.50 × 10% by weight;
(14) cyclo-methionyl-threonine or a salt thereof, content: 0.40 × 10⁻³ to 0.70 × 10% by weight;
(15) cyclo-alanyl-cysteine or a salt thereof, content: 0.40 × 10⁻³ to 0.70 × 10% by weight;
(16) cyclo-glycyl-cysteine or a salt thereof, content: 0.40 × 10⁻³ to 0.70 × 10% by weight;
(17) cyclo-aspartyl-serine or a salt thereof, content: 1.00 × 10⁻³ to 1.60 × 10% by weight;
(18) cyclo-arginyl-aspartic acid or a salt thereof, content: 0.90 × 10⁻³ to 1.50 × 10% by weight;
(19) cyclo-glycyl-tryptophan or a salt thereof, content: 0.10 × 10⁻³ to 0.30 × 10% by weight;
(20) cyclo-histidyl-phenylalanine or a salt thereof, content: 0.20 × 10⁻³ to 0.40 × 10% by weight;
(21) cyclo-aspartyl-leucine or a salt thereof, content: 0.40 × 10⁻³ to 0.80 × 10% by weight;
(22) cyclo-isoleucyl-histidine or a salt thereof, content: 0.70 × 10⁻³ to 1.20 × 10% by weight;
(23) cyclo-seryl-leucine or a salt thereof, content: 0.70 × 10⁻³ to 1.10 × 10% by weight;
(24) cyclo-isoleucyl-aspartic acid or a salt thereof, content: 0.70 × 10⁻³ to 1.10 × 10% by weight;
(25) cyclo-seryl-cysteine or a salt thereof, content: 0.40 × 10⁻³ to 0.70 × 10% by weight;
(26) cyclo-phenylalanyl-tryptophan or a salt thereof, content: 0.07 × 10⁻³ to 0.20 × 10% by weight;
(27) cyclo-alanyl-leucine or a salt thereof, content: 1.40 × 10⁻³ to 2.30 × 10% by weight;
(28) cyclo-glutaminyl-histidine or a salt thereof, content: 0.40 × 10⁻³ to 0.70 × 10% by weight;
(29) cyclo-arginyl-valine or a salt thereof, content: 0.60 × 10⁻³ to 1.00 × 10% by weight;
(30) cyclo-glutamyl-leucine or a salt thereof, content: 0.70 × 10⁻³ to 1.10 × 10% by weight;
(31) cyclo-leucyl-tryptophan or a salt thereof, content: 0.10 × 10⁻³ to 0.30 × 10% by weight;
(32) cyclo-tryptophanyl-tryptophan or a salt thereof, content: 0.04 × 10⁻³ to 0.07 × 10% by weight;
(33) cyclo-L-alanyl-proline or a salt thereof, content: 0.40 × 10⁻³ to 0.70 × 10% by weight;
(34) cyclo-methionyl-arginine or a salt thereof, content: 0.20 × 10⁻³ to 0.50 × 10% by weight;
(35) cyclo-lysyl-phenylalanine or a salt thereof, content: 1.00 × 10⁻³ to 1.60 × 10% by weight;
(36) cyclo-phenylalanyl-phenylalanine or a salt thereof, content: 0.30 × 10⁻³ to 0.60 × 10% by weight;
(37) cyclo-tryptophanyl-tyrosine or a salt thereof, content: 0.10 × 10⁻³ to 0.20 × 10% by weight;
(38) cyclo-asparaginyl-valine or a salt thereof, content: 0.30 × 10⁻³ to 0.50 × 10% by weight;
(39) cyclo-glutaminyl-isoleucine or a salt thereof, content: 0.50 × 10⁻³ to 0.80 × 10% by weight;
(40) cyclo-alanyl-serine or a salt thereof, content: 0.40 × 10⁻³ to 0.80 × 10% by weight;
(41) cyclo-methionyl-histidine or a salt thereof, content: 0.10 × 10⁻³ to 0.30 × 10% by weight;
(42) cyclo-methionyl-proline or a salt thereof, content: 0.10 × 10⁻³ to 0.30 × 10% by weight;
(43) cyclo-arginyl-leucine or a salt thereof, content: 1.50 × 10⁻³ to 2.30 × 10% by weight;
(44) cyclo-methionyl-glutamic acid or a salt thereof, content: 0.10 × 10⁻³ to 0.30 × 10% by weight;
(45) cyclo-methionyl-alanine or a salt thereof, content: 0.20 × 10⁻³ to 0.50 × 10% by weight;
(46) cyclo-isoleucyl-glutamic acid or a salt thereof: 0.50 × 10⁻³ to 0.90 × 10% by weight;
(47) cyclo-isoleucyl-serine or a salt thereof, content: 0.50 × 10⁻³ to 0.90 × 10% by weight;
(48) cyclo-valyl-serine or a salt thereof, content: 0.50 × 10⁻³ to 0.90 × 10% by weight;
(49) cyclo-methionyl-glycine or a salt thereof, content: 0.40 × 10⁻³ to 0.70 × 10% by weight;
(50) cyclo-valyl-threonine or a salt thereof, content: 0.30 × 10⁻³ to 0.60 × 10% by weight;
(51) cyclo-valyl-aspartic acid or a salt thereof, content: 0.50 × 10⁻³ to 0.90 × 10% by weight;
(52) cyclo-glycyl-proline or a salt thereof, content: 0.30 × 10⁻³ to 0.50 × 10% by weight;
(53) cyclo-leucyl-proline or a salt thereof, content: 0.50 × 10⁻³ to 0.90 × 10% by weight;
(54) cyclo-glutaminyl-glycine or a salt thereof, content: 0.05 × 10⁻³ to 0.09 × 10% by weight;
(55) cyclo-tryptophanyl-lysine or a salt thereof, content: 0.20 × 10⁻³ to 0.40 × 10% by weight;
(56) cyclo-glutaminyl-phenylalanine or a salt thereof, content: 0.30 × 10⁻³ to 0.60 × 10% by weight;
(57) cyclo-lysyl-glycine or a salt thereof, content: 1.00 × 10⁻³ to 1.60 × 10% by weight;
(58) cyclo-seryl-lysine or a salt thereof, content: 1.60 × 10⁻³ to 2.60 × 10% by weight;
(59) cyclo-valyl-lysine or a salt thereof, content: 0.90 × 10⁻³ to 1.50 × 10% by weight;
(60) cyclo-asparaginyl-lysine or a salt thereof, content: 0.60 × 10⁻³ to 1.10 × 10% by weight;
(61) cyclo-histidyl-histidine or a salt thereof, content: 0.40 × 10⁻³ to 0.70 × 10% by weight;
(62) cyclo-threonyl-histidine or a salt thereof, content: 0.40 × 10⁻³ to 0.70 × 10% by weight;
(63) cyclo-aspartyl-histidine or a salt thereof, content: 0.40 × 10⁻³ to 0.70 × 10% by weight;
(64) cyclo-asparaginyl-histidine or a salt thereof, content: 0.10 × 10⁻³ to 0.30 × 10% by weight;
(65) cyclo-arginyl-serine or a salt thereof, content: 0.40 × 10⁻³ to 0.70 × 10% by weight;
(66) cyclo-asparaginyl-methionine or a salt thereof, content: 0.40 × 10⁻³ to 0.70 × 10% by weight;
(67) cyclo-glutaminyl-methionine or a salt thereof, content: 0.10 × 10⁻³ to 0.20 × 10% by weight;
(68) cyclo-tryptophanyl-arginine or a salt thereof, content: 0.10 × 10⁻³ to 0.30 × 10% by weight;
(69) cyclo-asparaginyl-arginine or a salt thereof, content: 0.40 × 10⁻³ to 0.70 × 10% by weight;
(70) cyclo-asparaginyl-proline or a salt thereof, content: 0.05 × 10⁻³ to 0.09 × 10% by weight; and
(71) cyclo-arginyl-arginine or a salt thereof, content: 0.40 × 10⁻³ to 0.70 × 10% by weight.

9. The composition according to claim 7 or 8, wherein one or two or more of the following (1) to (10) as the cyclic dipeptide including amino acids as constituent units or salt thereof are each comprised in the following content range:
(1) cyclo-threonyl-tyrosine or a salt thereof, content: 0.40 × 10⁻³ to 0.70 × 10% by weight;
(2) cyclo-valyl-tyrosine or a salt thereof, content: 0.30 × 10⁻³ to 0.50 × 10% by weight;
(3) cyclo-glutaminyl-tyrosine or a salt thereof, content: 0.20 × 10⁻³ to 0.40 × 10% by weight;
(4) cyclo-asparaginyl-isoleucine or a salt thereof, content: 0.40 × 10⁻³ to 0.70 × 10% by weight;
(5) cyclo-arginyl-proline or a salt thereof, content: 0.40 × 10⁻³ to 0.70 × 10% by weight;
(6) cyclo-asparaginyl-glycine or a salt thereof, content: 0.20 × 10⁻³ to 0.50 × 10% by weight;
(7) cyclo-methionyl-valine or a salt thereof, content: 0.40 × 10⁻³ to 0.70 × 10% by weight;
(8) cyclo-glutamyl-cysteine or a salt thereof, content: 0.40 × 10⁻³ to 0.70 × 10% by weight;
(9) cyclo-seryl-glutamic acid or a salt thereof, content: 0.40 × 10⁻³ to 0.70 × 10% by weight; and
(10) cyclo-isoleucyl-lysine or a salt thereof, content: 0.50 × 10⁻³ to 0.90 × 10% by weight.

10. The composition according to any one of claims 1 to 9, wherein the cyclic dipeptide including amino acids as constituent units is derived from soybean.

11. The composition according to any one of claims 1 to 9, wherein the cyclic dipeptide including amino acids as constituent units is derived from tea.
